# EUROPEAN PATENT SPECIFICATION

(11) **EP 4 223 125 B1**
(45) Date of publication and mention of the grant of the patent: **09.12.2026**
(21) Application number: 23166377.4
(22) Date of filing: 24.09.2021
(51) Int. Cl.: A01N 63/14, A01K 67/36, A01P 7/02, A01P 7/04

(54) **METHODS AND COMPOSITIONS FOR REARING PREDATOR INSECTS**
VERFAHREN UND ZUSAMMENSETZUNGEN ZUR AUFZUCHT VON PRÄDATORINSEKTEN
PROCÉDÉS ET COMPOSITIONS POUR L'ÉLEVAGE D'INSECTES PRÉDATEURS

(30) Priority: 24.09.2020 EP 20382844
(43) Date of publication of application: 09.08.2023
(62) Document number(s) of the earlier application(s) in accordance with Art. 76 EPC: 21782739.3 / 4 195 933
(73) Proprietor: Agrobio S.L., 04745 El Viso (La Mojonera) - Almeria (ES)
(72) Inventor: VILA RIFÁ, Enrique, 04745 El Viso, La Mojonera, Almería (ES); BIELZA LINO, Pablo, 30203 Cartagena (ES)
(74) Representative: ABG Intellectual Property Law, S.L.

(56) References cited:
- BONTE JOCHEM ET AL: "Eggs of Ephestia kuehniella and Ceratitis capitata , and motile stages of the astigmatid mites Tyrophagus putrescentiae and Carpoglyphus lactis as factitious foods for Orius spp. : Factitious foods for Orius spp .", vol. 24, no. 4, 13 June 2016 (2016-06-13), United Kingdom, Taiwan, Republic of China, pages 613 - 622, XP055778697, ISSN: 1672-9609, Retrieved from the Internet <URL:https://api.wiley.com/onlinelibrary/tdm/v1/articles/10.1111%2F1744-7917.12293> [retrieved on 20210223], DOI: 10.1111/1744-7917.12293
- HUSSEINI M. ET AL: "Rearing immature feeding stage of Orius majesculus Reut. (Het., Anthocoridae) on the acarid mite Tyrophagus putriscentiae Schr. as new alternative prey", JOURNAL OF APPLIED ENTOMOLOGY, vol. 116, 12 January 1993 (1993-01-12), DE, pages 113 - 117, XP055779003, ISSN: 0931-2048
- BERNARDO A. M. G. ET AL: "Performance of Orius insidiosus on alternative foods", vol. 141, no. 9, 27 February 2017 (2017-02-27), DE, pages 702 - 707, XP055779021, ISSN: 0931-2048, Retrieved from the Internet <URL:https://api.wiley.com/onlinelibrary/tdm/v1/articles/10.1111%2Fjen.12390> [retrieved on 20210223], DOI: 10.1111/jen.12390

## Description

### FIELD OF THE INVENTION

The present invention belongs to the field of methods for rearing predator insects, in particular, insects from the family Anthocoridae, as well as methods for controlling crop pests using said insects.

### BACKGROUND OF THE INVENTION

There is an increasing demand to produce vegetables, fruits and ornamental plants using alternative non-chemical methods to control pests. One of the most important alternative methods is the use of the biological control offered by beneficial arthropods that are predators and/or parasitoids of these pests. This is recently named bioprotection of the plants. The possibility to develop and expand the use of biological control, specifically the use of arthropod natural enemies, is better than ever, with strong positive changes taking place in Europe. The EU Directive 2009/128/EC, together with the increasing demand by retailers and NGOs for products in which the level of pesticide residues is far below the national or international legal requirements, has been mainly responsible for this welcome upsurge in biological control usage. In the European Union, each country had to develop a National Action Plan (NAP) aimed at reaching the common objectives of this Directive, where Biological Control must be applied whenever possible.

The major development of biological control has occurred in greenhouses or plastic houses (protected crops). Biological control programs in greenhouses are often based on periodical releases of natural enemies, produced by biomanufacturers, also referred to as augmentative biological control. This method has been successfully applied for decades, and a professional bioindustry has emerged. However, in some cases there are shortcomings in pest control efficacy, which often can be attributed to the poor establishment of natural enemies. To overcome the problem of establishment and monitoring, some specialist natural enemies (e.g. aphid and whitefly parasitoids) are released routinely (e.g. weekly) as an "insurance policy". However, this method is not always economically viable.

A successful ratio is best, often only, achieved if a reasonable breeding population of the biocontrol agent can be established on the crop before the first pests invade. However, if predators are introduced before the pest arrives, they will quickly die of starvation, especially if the crop itself is unable to provide nutrients, such as pollen or nectar. Many valuable crops, e.g. cucumbers, do neither have flowers with pollen, nor possess nectar sites, and so do not have an alternative nutrient source. Other crops, such as young sweet pepper plants or ornamentals, will have yet to flower. However, even if pollen or flowers are present, not all predators can efficiently use pollen or nectar as food sources.

Thus, a major drawback with current biological programs for protected crops is that the factors which influence the arrival of the first pest populations are complex, and to date neither the commercial producer, nor the grower, has been able to reasonably identify the timing of this event.

Methods that will increase the persistence of natural enemies in crops could greatly enhance the efficacy, robustness and cost-effectiveness of biological pest control. The establishment and persistence of generalist predators compared to specialist natural enemies may provide more sustainable biological control, as their broader diet range enables them to persist or even reproduce on alternative prey or plant-provided food sources in the absence of pest organisms. This offers the opportunity to inoculate crops that provide such food sources with generalist predators before pest invasions (preventive biological control). However, many crops do not provide the additional resources required by natural enemies.

Polyphagous natural enemies include some species of Heteroptera and predatory mites of the family Phytoseiidae.

The introduction of pollen is one of the strategies that has been most investigated as a feeding system to favor the establishment of predatory mite populations, with highly controversial results. Some researchers conclude that pollen represents a viable proposition as an alternative pre-pest food, whereas others defer. This is probably not due to different approaches in methodologies but by the fact that there are very different qualities of "pollen". The pollen profile for each flowering plant is unique, thus to one predator species a specific pollen may represent a natural food, whilst to another it will be factitious, unknown to it in its natural environment. Other drawbacks associated with pollen as an alternative food are in themselves major problems, for example:
- pollen also provides a source of nutriment for many pest species
- scarcity of a regular pollen supply for commercial usage, relative to the need to employ different pollen species,
- reliability of purchasing from an outside source,
- storage problems relating to fresh pollen, chiefly rapid protein denaturing,
- currently only one commercial pollen product is on the market. This product is cattail pollen (*Typha* sp.). Thus, only a very small market can be satisfied, and
- in moist, warm environments, such as experienced by crops in certain countries, pollen will quickly become attacked by fungi.

Very successful strategies have been recently developed to improve establishment of predatory mites on the crops, based on the introduction, on top of the crop plants, of a group of prey mites as an alternative or complementary food. Specifically, a group of species of astigmatids, known as "stored food pest mites", can be reared in large numbers in heated chambers at reasonable costs. Programs and systems for the introduction of these prey mites have been established on top of crop plants, where they cannot produce any damage, as factitious prey for predatory mites. This has allowed improving the establishment of predatory mites and, especially, the development of biological control strategies in crops where it was previously difficult to establish these predators, as in the case of many ornamentals. In addition, it has been the key to the development of strategies in various open field crops, where the economic margins are, in general, lower and, therefore, the costs that a farmer can invest in inoculative releases of predators is much lower, compared to greenhouses.

However, a cost-effective system for establishing the other most widely used group of polyphagous predators, the zoophytophagous predator bugs, is still lacking. Among these, the bugs of the Miridae family are the most used for pest control in various crops, especially tomato and eggplant. The species that are massively released in greenhouses are *Macrolophus pygmaeus* in northern Europe, *Nesidiocoris tenuis* in Mediterranean countries and some Asians (South Korea) and Australia, and *Dicyphus hesperus* in Canada. These mirids have a longer period of development compared to most of the phytoseiid mites, and they need several weeks until establishment in the crop. Releases are mostly inoculative and pest control is only achieved subsequently by the following generations that develop in the crop, once populations are well established. For this reason, in order to favor the early establishment, a program of releases of *N*. *tenuis* on the nursery plants before transplanting were developed in the south of Spain. In these little seedling plants without pests or alternative nourishment, complementary food must be provided once or twice to support the predators. In other cooler areas, like Canada, Russia, east and north of Europe, where most of tomato crops are planted in winter in the greenhouses, the inoculative releases are made several weeks, or even months, after transplanting, when temperatures increase in spring. Then, an expensive feeding program, with weekly introductions of food for these predatory bugs during the first months, is also usually required to compensate for this delay.

Another problem is the drop of the populations when pests are at low levels, when temperatures are low (most of the plastic houses and tunnels have no heating system) and/or chemicals are applied to control diseases or some specific pests without biocontrol solutions. In these situations, biocontrol is disrupted and a cost/effective feeding program would help to overcome these problems.

The eggs of the moth *Ephestia kuehniella* is by far the best factitious food used, because they are considered a high-quality food, used in most of the commercial mass rearings. The efficacy of *E. kuehniella* eggs as "factitious prey" for many polyphagous predatory insects has been demonstrated in numerous studies and in some of them it has been even shown that these predators perform better than when fed on natural prey (Riddick, BioControl 2009, 54: 325-339). However, the use of *E. kuehniella* eggs as supplemental food is expensive, with a cost higher than 800 euros per kg, and this limits greatly its use. For this reason, several studies have been carried out to find an alternative food to *E. kuehniella* eggs.

The use of *Artemia* cysts as factitious prey for laboratory breeding of mirids has been evaluated. *Artemia* cysts are the encapsulated eggs of the brine shrimp *Artemia* spp., and when de-capsulated form a basic food for exotic ornamental fish species, hence large quantities are sold across the world each year. *Artemia* sp. cysts have been shown to be a good alternative food for rearing *M. pygmaeus* and *N. tenuis,* and its consumption allows preimaginal development and reproduction of the predator (Castañé et al. Biological Control 2006, 38: 405-412; Vandekerkhove et al. Journal of Applied Entomology, 2009, 133: 133-142). Fernandez Oveja (Control biológico en cultivos horticolas: efecto de los alimentos suplementarios en depredadores y parasitoides. PhD thesis. University of Lleida, Spain. http://hdl.handle.net/10803/293152, 2015) showed that *Artemia* allows to increase the fertility of *Macrolophus pygmaeus* and *N. tenuis,* and proposed their use as food to improve the establishment of these predators in the crop. However, the costs of *Artemia* are relatively high. Another problem is the variability of the quality of *Artemia.* This is the food most used for fish production in fish farms, being a market that consumes an enormous quantity, and there is currently a deficit in the quantity available. For this reason, the *Artemia* cysts that are supplied to feed predators in the crops are of low quality, reserving the best products for the fish farm market that can pay a higher cost.

Another group of polyphagous predators that has been the key to the success of augmentative biological control programs are the bugs of the Anthocoridae family. In particular, various species of the *Orius* genus, known as flower bugs, are inoculatively introduced into horticultural crops, such as bell peppers, to control thrips. Polyphagous predators *O*. *majusculus* and *O. laevigatus,* are two Paleartic species and two of the natural enemies widely used in biological control programs, both in inoculation and conservation strategies, for the control of thrips, especially the pest *Frankliniella occidentalis.* Like the mirids, these predators are polyphagous, also feeding on food of vegetable origin, especially pollen, and obtaining nutritional benefits from them (Pumariño and Alomar, Biological Control, 2012, 62(1): 24-28). Eggs of *Ephestia kuehniella* and *Ceratitis capitata* and motile stages of the astigmatid mites *Tyrophagus putrescentiae* and *Carpoglyphus lactis* have been assessed in the laboratory for their suitability as factitious foods for *Orius* spp. (Bonte et al., Insect Science, 2017, 24, 643-622).

*Orius laevigatus* is by far the most used species in Europe, it is released in all the sweet pepper crops as soon as flowering is above 50%, so individuals can feed on pollen as an alternative food. Nevertheless, it takes some time for predators to build up a significant population to cope with pests, especially feeding on a non-prey food like pollen. Therefore, an even earlier release would be very advantageous. In this sense, providing artificially with alternative food would allow predator release without the presence of prey or flowers. Moreover, innovations in varieties adapted to modern trends of healthy convenience food, like seedless baby peppers, bring about some side-effects for biocontrol, since these pepper plants do not produce pollen, hindering establishment of biological control agents, and so demanding application of supplemental food.

Additionally, during the crop cycle predator-prey populations interact and eventually the prey may disappear, and consequently the predator may also vanish due to a lack of prey. This situation would leave the crop without protection against immigrant pest populations. Presence of alternative food provided by the plant may reduce the impact on predator populations. However, the number of flowers and the amount and quality of pollen vary throughout the season. In addition, the same constrains that affect mirids are also limiting the success of anthocorids. Predator populations may drop due to a number of environmental and chemical stresses, such as extreme temperature and humidity. Also, in these cases, artificial supplementing of alternative food may help the recovery and persistence of the populations.

There are other crops without pollen, or without flowers when predators are introduced, where *O. laevigatus* can offer a successful control of pests, like cucumbers and ornamentals. Chrysanthemums, roses and other cut flowers are some of the crops deserving more attention, since the damage threshold of thrips is very low, and at present there are no solutions that guarantee a complete control. Several food applications are under evaluation by researchers to improve the establishment of *O. laevigatus* in these protected crops.

Therefore, there is a need for new methods for rearing predator insects on crops in the absence of their natural prey and even in the absence of pollen.

### SUMMARY OF THE INVENTION

The authors of the present invention have found that, surprisingly, insects from the families Miridae or Anthocoridae, which are natural predators for common crop pests, are capable to feed on crops on astigmatid mites, a factitious prey for these predators (see Examples 1, 2, 3). In some cases, the predator insects performed better when fed on astigmatid mites than when fed on other expensive factitious prey like cysts of *Artemia* or eggs of *Ephestia kuehniella* (see Examples 2 and 3). Therefore, astigmatid mites can be used to feed these predator insects directly on the crop as a part of a biological control strategy, resulting in a successful control of the pests (see Example 2). Moreover, the inventors have selected a strain of the insect *Orius laevigatus* with an increased ability to develop and reproduce when fed on pollen (Example 4) and have shown that, surprisingly, this new strain has also an increase tolerance to factitious prey, in particular, astigmatid mites (see Examples 5, 6 and 7).

Therefore, in a first aspect, the invention relates to a method for rearing a predator insect from the genus *Orius* on a crop comprising:
(a) providing the plant with at least a breeding population of the insect from the genus *Orius,*
(b) providing the plant with a first source of food for the predator insect, wherein said first source of food comprises a population of an astigmatid mite, wherein said population of an astigmatid mite is not a population of *Tyrophagus putrescentiae*
   and
(c) allowing the predator insect to prey on the astigmatid mite,
wherein the astigmatid mite is a storage mite from a family selected from the group consisting of Acaridae, Carpoglyphidae, Glycyphagidae and Suidasiidae.

In a second aspect, the invention relates to a method for controlling a pest in a crop comprising:
(a) providing the crop with at least a breeding population of an insect from the genus *Orius,* wherein said insect is a predator for said pest and
(b) providing the crop with a first source of food for the predator insect, wherein said first source of food comprises a population of an astigmatid mite, wherein said population of an astigmatid mite is not a population of *Tyrophagus putrescentiae*
wherein the astigmatid mite is a storage mite from a family selected from the group consisting of Acaridae, Carpoglyphidae, Glycyphagidae and Suidasiidae.

In a third aspect, the invention relates to a use of an astigmatid mite for rearing a predator insect from the genus *Orius* on a crop, wherein said astigmatid mite is not *Tyrophagus putrescentiae,* wherein the astigmatid mite is a storage mite from a family selected from the group consisting of Acaridae, Carpoglyphidae, Glycyphagidae and Suidasiidae.

In another aspect, the invention relates to a composition comprising at least a breeding population of a predator insect from the genus *Orius* and a first source of food for said predator insect, wherein said first source of food comprises a population of an astigmatid mite, wherein said population of an astigmatid mite is not a population of *Tyrophagus putrescentiae* nor a population of *Carphoglyphus lactis,* wherein the breeding population of the predator insect and the first source of food are physically separated so that there is no contact between the population of the predator insect and the population of the astigmatid mite, and wherein the astigmatid mite is an storage mite from a family selected from the group consisting of Acaridae, Carpoglyphidae, Glycyphagidae and Suidasiidae.

In another aspect, the invention relates to the use of the composition according to the previous aspect for controlling a pest in a plant, where the pest is a prey for the predator insect.

### BRIEF DESCRIPTION OF THE FIGURES

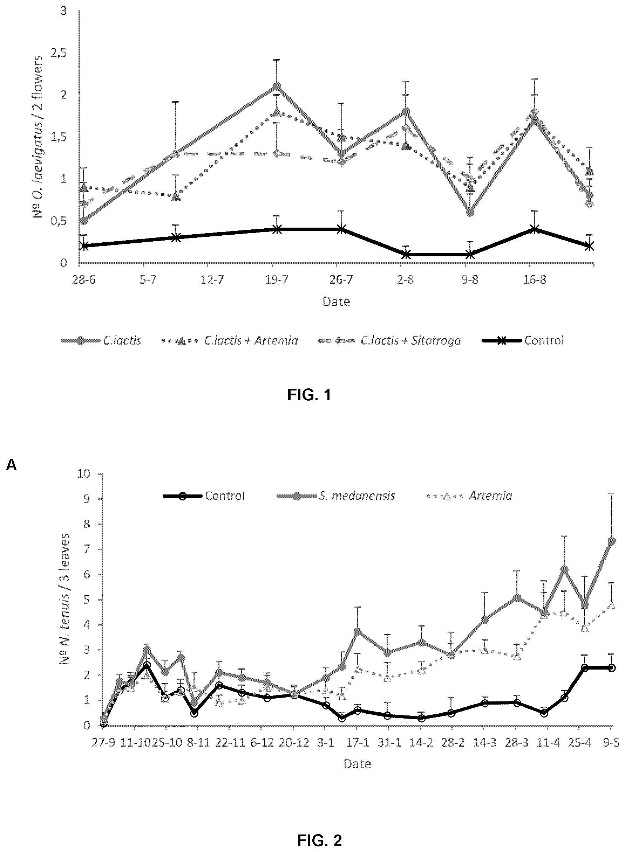

**Figure 1.** Mean (±SE) of nymphs and adults of *O. laevigatus* counted between June and August 2019 in a commercial crop. A pepper crop of a variety type mini Capia was planted in May the 10^{th} and 4 treatments were established according to the factitious prey introduced: (1) prey mite *C. lactis,* (2) *C. lactis* + cysts of *Artemia,* (3) *C. lactis +* eggs of *S. cerealella* and (4) control without factitious prey.

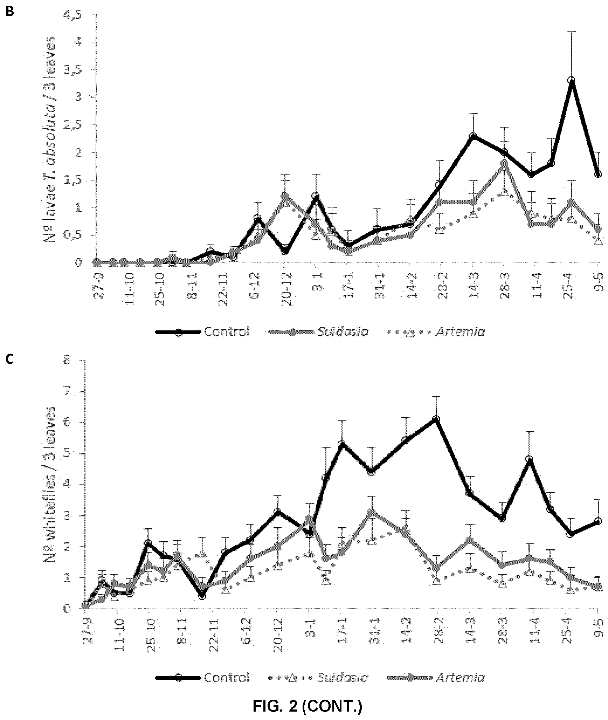

**Figure 2****.** Mean (±SE) of nymphs and adults of *N. tenuis* (a), larvae of *Tuta absoluta* (b) and nymphs, pupae and adults of whiteflies (c) counted between September 2017 and May 2018 in a commercial greenhouse. A tomato crop of a variety Genio was planted in August the 23rd and 3 treatments were established according to the factitious prey introduced: (1) prey mite *S. medanensis,* (2) cysts of *Artemia* and (3) control without factitious prey.

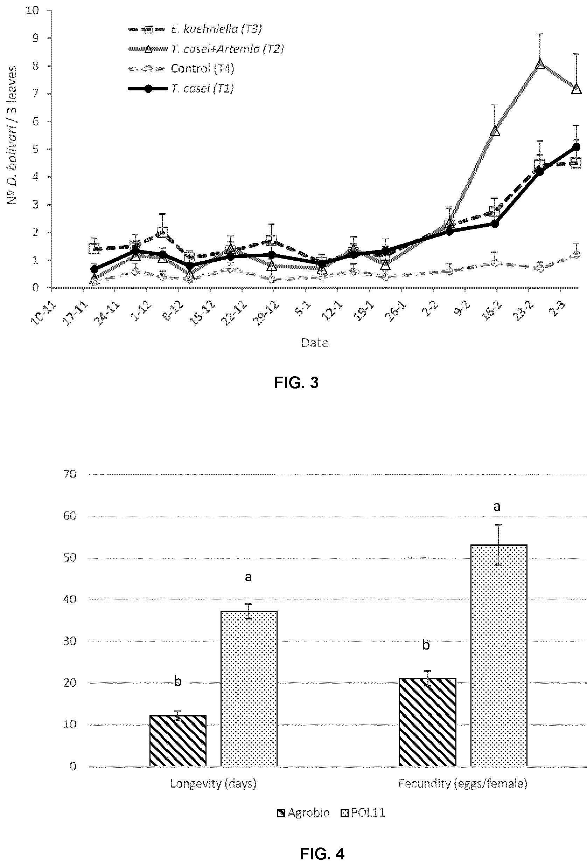

**Figure 3****.** Mean (±SE) of nymphs and adults of *D*. *bolivari* counted between November 2018 and March 2019 in an experimental greenhouse. A tomato crop of a variety Caniles was planted in October the 31^{st}, and 4 treatments were established according to the factitious prey introduced: (1) prey mite *T. casei,* (2) *T. casei* complemented with cysts of *Artemia,* (3) eggs of *E. kuehniella* and (4) control without factitious prey.
**Figure 4****.** Longevity and lifetime fecundity (mean ± SE) of females of a commercial population (Agrobio) of *O*. *laevigatus* and a new strain (POL11) feeding Astigmatid mites.

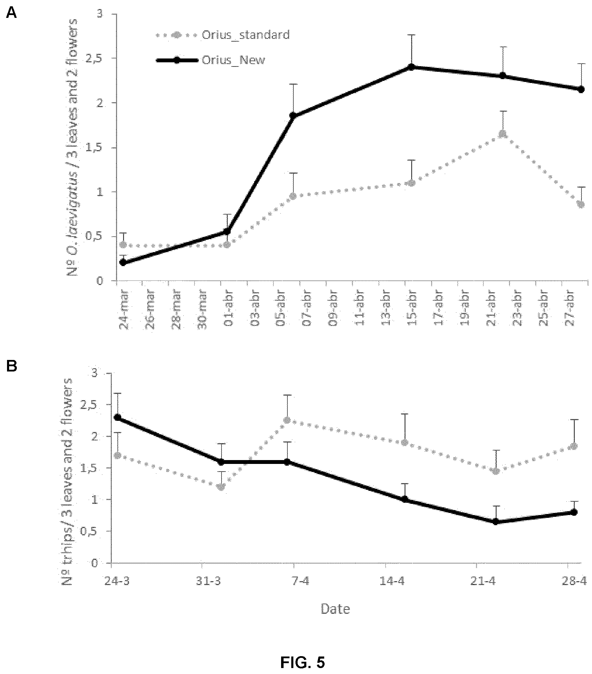

**Figure 5****.** Mean (±SE) of nymphs and adults of *O*. *laevigatus* (a) and larvae and adults of thrips (b) counted between March and April 2020 in a commercial crop. A pepper crop of the variety mini Capia 'Pepperito' was planted in February the 2^{on} and 2 treatments were established: (1) releases of a new strain of *O*. *laevigatus* and (2) releases of standard commercial populations of *O*. *laevigatus.*

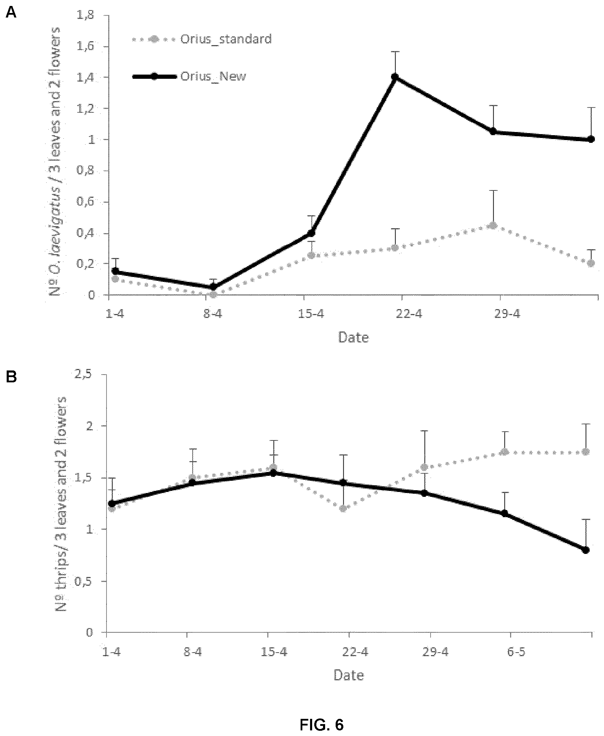

**Figure 6****.** Mean (±SE) of nymphs and adults of *O*. *laevigatus* (a) and larvae and adults of thrips (b) counted between April and May 2020 in a commercial crop. A Dutch type cucumber crop was planted in February the 27th and 2 treatments were established: (1) releases of a new strain of *O*. *laevigatus* and (2) releases of standard commercial populations of *O*. *laevigatus.*

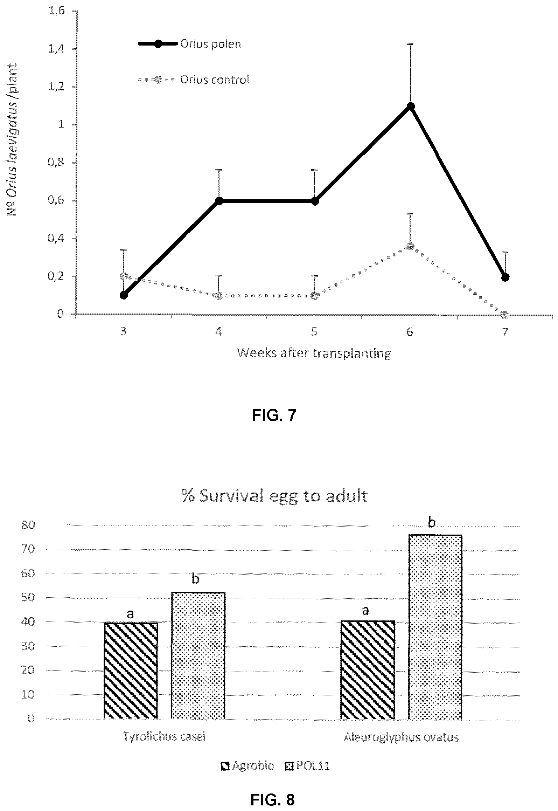

**Figure 7****.** Mean (±SE) of nymphs and adults of *O*. *laevigatus* counted between February and March 2020 in a commercial crop. A chrysanthemums crop was planted in January the 28th and 2 treatments were established: (1) releases of a new strain of *O*. *laevigatus* and (2) releases of standard commercial populations of *O*. *laevigatus.*
**Figure 8****.** Survival from egg to adult of females of a commercial population (Agrobio) and a new strain (POL11) feeding two Astigmatid mite species. Bars with the same letter within each mite species are not significantly different (p>0.05).

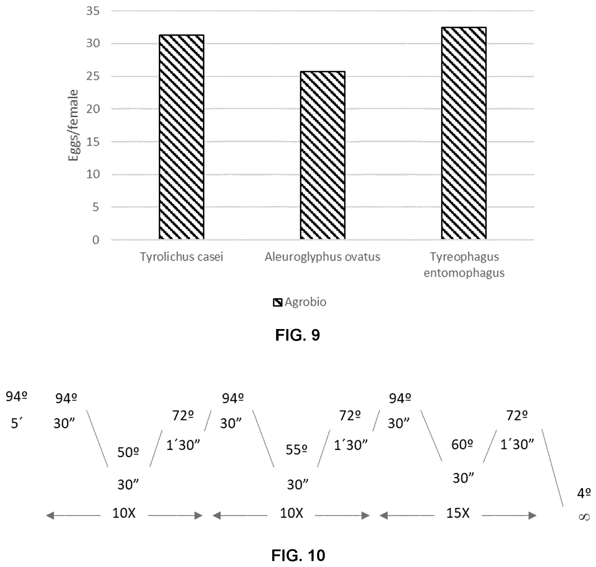

**Figure 9****.** Lifetime fecundity of females of a commercial population (Agrobio) feeding different Astigmatid mite species.
**Figure 10****.** PCR program used for amplification of the SSR marker RP02.

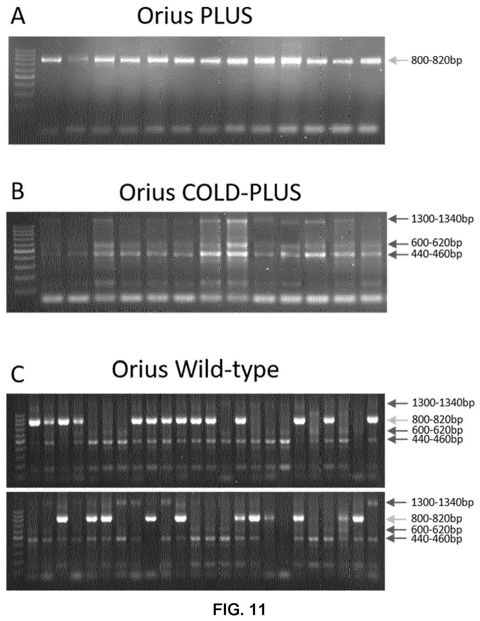

**Figure 11****.** Electrophoresis images from PCR amplification with RP02 SSR marker in 13 adults of each improved strain, Orius PLUS (A), Orius COLD-PLUS (B) and in 48 individuals from a wild population, Orius Wild-type (C). In line 1 is placed 100bp DNA Ladder. The ladder consists of fragments ranging in size from 100-1.000 bp in 100 bp increments.

### DETAILED DESCRIPTION OF THE INVENTION

### Method for rearing a predator insect

In a first aspect the invention relates to a method for rearing a predator insect from the genus *Orius* on a crop comprising:
(a) providing the plant with at least a breeding population of the insect from the genus *Orius,*
(b) providing the plant with a first source of food for the predator insect, wherein said first source of food comprises a population of an astigmatid mite,
   and
(c) allowing the predator insect to prey on the astigmatid mite,
wherein the astigmatid mite is a storage mite from a family selected from the group consisting of Acaridae, Carpoglyphidae, Glycyphagidae and Suidasiidae.

The term "rearing", as used herein, broadly refers to breeding, reproducing, surviving and growing of individuals, and includes the propagation and increase of a population by sexual reproduction.

The first method of the invention is directed to rear a predatory insect from the genus *Orius* on a crop. The expression "rearing on a crop", as used herein, means that these predatory insects are located on the surface of any plant, including pot plants and crops, including an open field crop or on a greenhouse crop. The term "crop", as used herein, refers to a plant of economic importance and/or a men-grown plant.

In a particular embodiment, the plant or crop is selected from the group consisting of vegetables (including cucumber, pepper, eggplant, zucchini, melon, watermelon, tomato, leaf vegetables like lettuce or rocket, potato, beans and onion), orchards, vineyard, olive trees, citrus trees, stone fruit trees, berries, ornamental plants, including pot plants and cut flowers, aromatic plants, and plants for pharmaceutic use like cannabis. In a more particular embodiment, the plant or crop is selected from the group consisting of pepper, tomato, cucumber and chrysanthemums.

The term "pepper" refers to a plant of the genus Capsicum, identified in the NCBI database by the Taxonomy ID: 4071. The term "pepper" or "Capsicum" includes any species, for example. In a particular embodiment, the pepper crop is from the type "Capia" and from the variety "Pepperito".

The term "tomato" refers to a plant of the species *Solanum lycopersicum,* identified in the NCBI database by Taxonomy ID: 4081.The term "tomato" or *"Solanum lycopersicum"* includes any tomato variety. In a particular embodiment, the tomato crop is from the variety "Genio" (from Clause) or from the variety "Caniles".

The term "cucumber" refers to a plant of the species *Cucumis sativus,* identified in the NCBI database by Taxonomy ID: 3659. The term "cucumber" or *"Cucumis sativus"* includes any variety. In a particular embodiment, the cucumber is from the Dutch type variety Litoral (Rijk Zwaan).

The term "family Miridae", as used herein, refers to a family of insects belonging to the suborder Heteroptera commonly known as capsid bugs or mirid bugs. The family Miridae corresponds to the family identified in the NCBI database by the Taxonomy ID: 30083. Most widely known mirids are species that are notorious agricultural pests. However, some species are predatory.

The term "predatory insect", as used herein, refers to an insect that eats other animals, called prey. Predator insects from the family Miridae (also known as "predatory mirids") are considered zoophytophagous or true omnivore predators, as these organisms consume both plant and animal resources during their life cycle. Predatory mirids are important predators for plant pests, like whiteflies, thrips, aphids, leafminers, spidermites and lepidoptera.

Illustrative non-limitative examples of predatory insects from the family Miridae includes insects from the subfamilies Bryocorinae, Deraeocorinae, Phylinae and Orthotylinae, preferably, from the subfamily Bryocorinae and the tribe Dicyphini, from the subfamily Deraeocorinae and the tribe Deraeocorini, from the subfamily Phylinae and the tribes Pilophorini and Nasocorini, from the subfamily Orthotylinae and the tribe Orthotylini. More preferably, the insects from the tribe Dicyphini are selected from the genus Nesidiocoris, such as *N. tenuis, N. volucerand N. callani*, the genus *Macrolophus,* such as *M pygmaeus* (formerly described as *M. caliginosus*), *M. melanotoma, M. costalis* and *M. basicornis,* the genus *Engytatus,* such as *E. varians* and E. *modestus,* from the genus *Dicyphus,* such as *D*. *tamaninii, D. bolivari, D. errans, D. hesperus* and *D*. *marrocanus,* from the genus *Tupiocoris,* such as *T. cucurbitaceus,* from the genus *Campyloneuropsis,* such as *C*. *infumatus,* or from the genus *Cyrtopeltis,* such as *C. callosus.* The insects selected from the tribe Deraecorini are preferably selected from the genus *Deraeocoris*, such as *D*. *brevis* and *D*. *nebulosus,* the insects selected from the tribe Pilophorini are preferably selected from the genus *Pilophorus,* such as *P. typicus* and *P. gallicus.* The insects selected from the tribe Nasocorini are preferably from the genus *Campylomma,* such as *C. verbasci* and *C. chinensis.* The insects selected from the tribe Orthotylini are preferably from the genus *Cyrtorhinus, Orthotylus* and *Eurotas,* such as *Cyrtorhinus lividipennis, Orthotylus marginalis* and *Eurotas brasilianus.*

In a particular aspect not encompassed by the claimed invention, the predator insect from the family Miridae is from a genus selected from the group consisting of *Macrolophus, Nesidiocoris, Dicyphus, Deraeocoris*, *Engytatus, Tupiocoris, Campyloneuropsis, Cyrtopeltis, Pilophorus, Campylomma, Cyrtorhinus, Orthotylus* and *Eurotas.* In a more particular embodiment, the predator insect from the family Miridae is from a species selected from the group consisting of *Macrolophus pygmaeus, M. costalis, M. basicornis, Nesidiocoris tenuis, N. volucer, N. callani, Dicyphus bolivari., D. errans, D. hesperus, D. marrocanus, D. geniculatus, D. tamaninii, Engytatus varians, E. modestus, Tupiocoris cucurbitaceus, Campyloneuropsis infumatus, Cyrtopeltis callosus, Deraeocoris brevis, D. nebulosus, Pilophorus typicus, P. gallicus, Campylomma verbasci, C. chinensis, Cyrtorhinus lividipennis, Orthotylus marginalis* and *Eurotas brasilianus.*

In a particular aspect not encompassed by the claimed invention, the predator insect from the family Miridae is from the genus *Macrolophus, Nesidiocoris* or *Dicyphus,* more particularly from the species *Nesidiocoris tenuis, M. pygmaeus, D. hesperus* or *Dicyphus bolivari.*

The term "family Anthocoridae", as used herein, refers to a family of insects belonging to the order Hemiptera and the superfamiliy Cimicoidea, and are commonly known as minute pirate bugs or flower bugs. The family Anthocoridae corresponds to the family identified in the NCBI database by the Taxonomy ID: 82738. Insects from the family Anthocoridae can feed on plants, but are mostly predatory, feeding on other small-soft bodied arthropods, some of which can be agricultural pest, such as mites and thrips.

Illustrative non-limitative examples of predatory insects from the family Anthocoridae include the tribe Anthocorini, Orinii and Xylocorini. Preferably, the insects from the Anthocorini are selected from the genus *Anthocoris,* such as *A. nemorum, A. nemoralis, A. confusus* and *A. minki,* the genus *Wollastoniella,* such as *W. rotunda,* and the genus *Blaptostethus,* such as *B. pallescens,* the insects from the tribe Orinii are selected from the genus *Orius,* such as *O. laevigatus, O. insidiosus, O. majusculus, O. niger, O. albidipennis, O. minutus, O. thripoborus, O. naivashae, O. strigicollis, O. sauteri, O. tristicolor, O. nagaii, O. tantillus, O. limbatus, O. thripoborus, O. naiashae, O. horvathi, O. vicinus, O. pumilio, Orius laticollis,* and *O. lindbergi,* the genus *Montandoniola,* such as *M. confusa* and *M. pictipennis,* and the genus *Macrotracheliella,* and the insects from the tribe Xylocorini are preferably selected from the genus *Xylocoris,* such as *X. flavipes.*

In a particular aspect not encompassed by the claimed invention, the predator insect from the family Anthocoridae is an insect from a genus selected from the group consisting of *Orius, Anthocoris, Blaptostethus, Montandoniola* and *Xylocoris.*

In a particular embodiment, the predator insect from the genus *Orius* is an insect from a species selected from the group consisting of *Orius laevigatus, O. insidiosus, O. majusculus, O. niger, O. albidipennis, O. minutus, O. thripoborus, O. naivashae, O. strigicollis, O. sauteri, O. tristicolor, O. nagaii, O. antillus, O. limbatus, O. thripoborus, O. naiashae, O. horvathi, O. vicinus, O. pumilio, Orius laticollis and O. lindbergi.*

In a more particular embodiment, the predator insect from the genus *Orius* is *Orius laevigatus. Orius laevigatus* corresponds to a species identified in the NCBI database by the Taxonomy ID: 82742.

In an even more particular embodiment, the predator insect from genus *Orius* is an insect from a strain from the species *Orius laevigatus* with increased tolerance to a diet based on a factitious food, wherein said strain is obtained by the method for obtaining a strain with increased tolerance to a diet based on a factitious food of the invention.

In another particular embodiment, the predator insect from the genus *Orius* is an insect from a strain from the species *Orius laevigatus* with increased tolerance to a diet based on a factitious food, wherein said strain is characterized in that when the genome of the strain is amplified by PCR using the pair or primers of SEQ ID NO: 1 and SEQ ID NO: 2 the following fragments are obtained in at least 70% of the individuals of the strain:
(a) a fragment of 780-840 base pairs or
(b) a fragment of 1280-1360 base pairs, a fragment of 580-640 base pairs and a fragment of 420-480 base pairs.

This strain is fully defined below.

In a particular embodiment, the fragment of 780-840 base pairs has 800-820 base pares.

In a particular embodiment, the fragment of 1280-1360 bp has 1300-1340 bp, more particularly 1300 bp.

In a particular embodiment, the fragment of 580-640 bp has 600-620 bp, more particularly 600 bp.

In a particular embodiment, the fragment of 420-480 bp has 440-460 bp, more particularly 450 bp.

In another particular embodiment, the predator insect is from a strain with increased tolerance to a diet based on a factitious food, wherein said strain is obtained by the methods for obtaining a strain with increased tolerance to a diet based on a factitious food of the invention.

The first method of the invention comprises a step of providing the plant with a breeding population of the predator insect from the genus *Orius.*

The term "breeding population", as used herein, refers to a population that can increase their number by sexual reproduction. A breeding population can comprise alive sexually mature individuals of both sexes and/or other stages of life, for example, eggs and/or nymph from both sexes that can mature into sexually mature adults. Alternatively, a breeding population can comprise one or more fertilized females.

The term "adult", as used herein referred to the predator insect, refers to an individual sexually mature or imago. It is in the adult stage that insects reproduce. In biology, the imago is the last stage an insect attains during its metamorphosis, its process of growth and development; it also is called the imaginal stage, the stage in which the insect attains maturity. It follows the final ecdysis of the immature instars. In a member of the Ametabola or Hemimetabola, in which metamorphosis is "incomplete", like the predatory bugs from the family Miridae or Anthocoridae, the final ecdysis follows the last immature or *nymphal* stage.

The term "nymph", as used herein referred to the predator insect, refers to an individual sexually immature, similar to the adult and found in such insects which have incomplete, or hemimetabolic, metamorphosis. Wings, if present, develop from external wing buds after the first few molts. The body proportions of the first nymphal stages are quite different from those of the adult. During each successive growing stage (instar) the nymph begins to resemble the adult more closely. The predatory Mirids and Anthocorids have 5 nymphal instars before reaching the adult stage.

The term "egg", as used herein referred to the predator insect, refers to the first stage of the insects. Eggs of Miridae and Anthocoridae generally are moderate to large in size relative to adults and have a smooth to finely sculptured surface, sometimes with a colour pattern and often with slender projections. Eggs contain sufficient nutrients to permit the embryo to develop into a free-living, sexually immature, wingless nymph. Predatory mirids and anthocorids generally insert the eggs into the tissue of selected host plants.

When nymph populations are supplied the last stages 4^{th} and 5^{th} are preferred. Additionally, substrates containing eggs can optionally be introduced, either with nymphs, adults or mixtures. Preferably pieces of vegetables, for instance beans, with the eggs embedded into the tissue, but also some artificial substrates containing the eggs can be introduced. The pieces of vegetables have the advantage that are also a source of water and nutrients for the predators.

The introduction of adults containing females that are ready to lay eggs is often preferred in order to fasten the establishment of the populations in the crop. In a particular embodiment, the breeding populations comprises between 30 and 100% of females, preferably between 40 and 80%, more preferably between 45 and 65%. The females need a preoviposition period to start laying eggs, which may vary according to the species. In a particular embodiment, the breeding population comprises adults between 1 hour and 10 days old. The age of the adults refers to the time elapsed since molting. The needed transport time from the mass-rearing facilities to the crop is also conditioning the age of the females when released. For this reason, the released adults are preferably between 1 and 7 days old, more preferably between 2 and 6 days old, most preferably between 3 and 5 days old. In a particular embodiment, the released adults are between 3-5 days if the adults have been maintained at a temperature comprised between 8°C and 20°C and between 2-4 days if the adults have been maintained at a temperature between 20°C and 30°C.

Predators are introduced into the plant or crop preferably with a carrier material. This facilitates the distribution of loose material containing the populations. The carrier material or substrate is also commonly necessary to minimize the mortality produced by cannibalistic behavior of the living predators during storage and transport from the rearing facilities to the crop. Substrates can be any material that provides a surface where predators can hide from each other. Preferably, the materials contain cavities where the predators can hide. Examples of substrates are cardboard or wrinkled paper, sawdust, shells of seeds, for example buckwheat, popcorn, and vermiculite. However, populations can also be supplied without substrate and, optionally, they can be mixed with a carrier material after transport, just before the distribution in the crop.

Predators are introduced on the plants before or after transplanting the crop or both. Preferably, the predators are introduced between 15 days and 1 hour before transplanting the crop when a fast establishment is desired. More preferably the predators are introduced between 12 days and 1 day before transplanting, and even more preferably between 10 days and 7 days before transplanting. When predators are introduced before transplanting, between 1 and 4 introductions are performed, preferably between 1 and 3, most preferably between 1 and 2 introductions. When predators are introduced after transplanting, preferably between 1 and 30 introductions are performed, more preferably between 1 and 10 introductions, most preferably between 1 and 4 introductions.

A ratio of between 0.05 and 50 individuals (nymphs, adults or mixtures) per plant are introduced each releasing time. Preferably, between 0.08 and 10 individuals per plant are introduced, more preferably between 0.3 and 5, and even more preferably between 0.5 and 2 individuals per plant. Ratios depend on the target crop, with the lowest numbers per plant introduced on crops with high density of plants, for instance, in cut flowers like chrysanthemums, where typically transplanting is performed at more than 60 plants per m². The highest numbers are introduced on crops with low density and bigger plants, for instance orchards.

Since commercial crop patterns, especially 'rowed' crops, as well as plant configurations can limit the spread of the predator which is essential for good control, in a preferred embodiment of the first method of the invention, the population of the predator insect is released in the plant or crop at a plurality of 'spots'. The density of releasing spots of predators in the crop is preferably between 1000 spots/m² and 1 spot/3500 m². However, even higher densities of spots in the crop are possible, especially when the material is sprayed by machinery in the seedlings before transplanting. When the populations are introduced in the crop after transplanting, more preferably the material is spread by hand into cardboard cages that are hang on the plants, being considered each cage a spot or releasing point. Then, the preferred density is between 1 releasing spot/ 5 m² and 1 spot/ 2000 m², most preferably between 1 spot/ 10m² and 1 spot/100 m², and even more preferably between 1 spot / 12m² and 1 spot / 25 m².

To provide a suitable density of predators and to establish a stable population of predators it is preferred that a population comprising between 1 and 1000 individuals of the predatory insects is released at each releasing spot, preferably between 5 and 500 individuals, more preferably between 10 and 100 individuals, most preferably between 25 and 50 individuals of the predatory insects. The number of individuals can be adjusted depending on the required density of the releasing spots in the crop, the activity of the predatory insects, the species of predator insects and their reproductive rate.

The first method of the invention comprises a step of providing the plant with a first source of food for the predator insect, wherein said first source of food comprises a population of an astigmatid mite, wherein said population of an astigmatid mite is not a population of *Tyrophagus putrescentiae* and wherein the astigmatid mite is a storage mite from a family selected from the group consisting of Acaridae, Carpoglyphidae, Glycyphagidae and Suidasiidae.

The term "astigmatid mite", as used herein, refers to a mite belonging to the Astigmata. The term "Astigmata", as used herein, refers to the infraorder Astigmata, belonging to the suborder Sarcoptiformes and the order Acariformes. In the past Astigmata was classified as an Order, but recently a modification to infraorder was proposed by Mironov & Bochkov, Entomological Review, 2009, 89 (8): 975-992. Astigmata is identified in the NCBI database by the Taxonomy ID: 6951.

The term "population of an astigmatid mite", as used herein, refers to a group of individuals that can include individuals of both sexes and of any stages of life, for example, adults and/or nymphs and/or eggs. The population of the astigmatid mite can be, a breeding population as previously defined, but it can also comprise dead individuals or can even be formed in its entirety by dead individuals. In a particular embodiment, at least part of the population of the population of the astigmatid mite is not alive, for example, at least a 5 %, at least a 10%, at least a 20%, at least a 30%, at least a 40%, at least a 50%, at least a 60 %, at least a 70%, at least a 80%, at least a 90% or the 100% of the population. In a particular embodiment, at least part of the population of the astigmatid mite is dead by a human action, for example, radiation exposure, freezing or exposure to high carbon dioxide levels.

The astigmatid mite is a storage food mite. The term "storage food mite", refers to astigmatid mites that feed on stored food. These mites can be found in environments where there is moisture or increased humidity, but are most frequently found in dry food items such as flour, grains, dried fruits, cereal and dry dog and cat foods. Illustrative non-limitative examples of storage food mites include the species described as storage food mites by Hughes, (1976), in her book entitled - 'The mites of stored foods and houses' (2nd edition, London. Ministry of Agriculture, Fisheries and Food, Technical Bulletin 9, iv, 400 p). This author describes some 30 species which qualify as belonging to the group known as 'stored food, or 'storage mites'. They represent just six families and about 18 genera. It is from this group, that all astigmatid species used as factitious prey for phytoseiid predatory mites have been selected until present. In a particular embodiment, the storage food mite is selected from the group consisting of *Acarus siro, A. farris, A. immobilis, Lardoglyphus konoi, Aleuroglyphus ovatus, Mycetoglyphus fungivorus, Tyrolichus casei, Tyrophagus tropicus, T. brevicrinatus, T. putrescentiae, T. neiswanderi, T. longior, T. similis, T. palmarum, T. perniciosus, Tyroborus lini, Rhizoglyphus callae, R. robini, Tyreophagus entomophagus, Caloglyphus berlesei, C. mycophagus, C. rhizoglyphoides, C. oudemansi, C. hughesi, C. claparède, Suidasia nesbitti, S. medanensis, Blomia freemani, Glycyphagus domesticus, G. privatus, G. ornatus, G. bicaudatus, Lepidoglyphus fustifer, L. destructor, Austroglycyphagus geniculatus, Diamesoglyphus intermedius, Ctenoglyphus palmifer, C. plumiger, C. canestrinii, Gohieria fusca Carpoglyphus munroi, C. lactis and Carpoglyphus wardleorum.*

In a more particular embodiment, the storage food mite is from a genus selected from the group consisting of *Acarus, Tyrophagus, Aleuroglyphus, Caloglyphus, Suidasia, Thyreophagus, Carpoglyphus, Glycyphagus, Tyrolichus, Tyroglyphus Lepidoglyphus* and *Blomia .*

In an even more particular embodiment, the storage food mite is from a species selected from the group consisting of *Suidasia medanensis, Suidasia nesbitti, Lepidoglyphus (Glycyphagus) destructor, Blomia freeman, Carpoglyphus lactis, Carpoglyphus munroi, Carpoglyphus wardleorum, Aleuroglyphus ovatus, Tyrolichus casei, Thyreophagus entomophagus* and *Acarus siro.*

In a particular embodiment, the storage food mite is from a family selected from the group consisting of Acaridae, Carpoglyphidae and Suidasiidae, more particularly from a genus selected from the group consisting of *Carpoglyphus, Suidasia, Tyreophagus, Aleuroglyphus* and *Tyrolichus,* even more particularly from a species selected from the group consisting of *Carpoglyphus lactis, Aleuroglyphus ovatus, Suidasia medanensis, Tyreophagus entomophagus and Tyrolichus casei.*

The term *"Carpoglyphus lactis",* as used herein, refers to a species identified in the NCBI database by the Taxonomy ID: 223459.

The term *"Suidasia medanensis",* as used herein, refers to a species identified in the NCBI database by the Taxonomy ID: 2236625.

The term *"Tyrolichus casei*", as used herein, refers to a species identified in the Global Biodiversity Information Facility database by the Taxonomy ID: 2181934.

The term '*Tyreophagus entomophagus',* as used herein, refers to a species in the Global Biodiversity Information Facility database by the Taxonomy ID: 2181946.

The term '*Aleuroglyphus ovatus',* as used herein, refers to a species in the Global Biodiversity Information Facility database by the Taxonomy ID: 5860704.

In a particular embodiment, the astigmatid mite is not an astigmatid mite that has plants as its natural habitat or that is harmful to agricultural crops. Illustrative non-limitative examples of astigmatid mites that have plants as their natural habitat and/or are harmful to agricultural plants include *Tyrophagus putrescentiae, Tyrophagus neiswanderi, Tyrophagus similis, Tyrophagus perniciosus, Tyrophagus robertsoni, Tyrophagus longior, Acarus farris, Rhizoglyphus echinopus, R. robini, Caloglyphus sp., Mycetoglyphus fungivorus* or *Schwiebea sp.* In a particular embodiment, the population of the astigmatid mite is not a population of *Tyrophagus neiswanderi, Tyrophagus similis, Tyrophagus perniciosus, Tyrophagus robertsoni, Tyrophagus longior, Acarus farris, Rhizoglyphus echinopus, R. robini, Caloglyphus mycophagus, Mycetoglyphus fungivorus* or *Schwiebea sp.* In another particular embodiment, the population of the astigmatid mite is not a living population of *Tyrophagus neiswanderi, Tyrophagus similis, Tyrophagus perniciosus, Tyrophagus robertsoni, Tyrophagus longior, Acarus farris, Rhizoglyphus echinopus, R. robini, Caloglyphus mycophagus, Mycetoglyphus fungivorus* or *Schwiebea sp.*

In a particular embodiment, the population of the astigmatid mite is not a population of *Tyrophagus.*

In a particular embodiment, the population of the astigmatid mite is not a population of *Carpoglyphus lactis.*

The term "living population", as used herein, refers to a population where substantially all individuals are alive, or at least 75%, at least 80%, at least 85%, at least 90 %, at least 95%, at least 96%, at least 97%, at least 98%, at least 99% of the individuals are alive.

The term *"Tyrophagus putrescentiae",* as used herein, refers to a species identified in the NCBI database by the Taxonomy ID: 59818.

The fist source of food for the predator insect can comprise more than one population of an astigmatid mite, for example 2, 3, 4, or more populations of different astigmatid mites. In a particular embodiment, the first source of food comprises two populations of different astigmatid mites, more particularly, two populations selected from the group consisting of *Acarus, Aleuroglyphus, Suidasia, Tyreophagus, Carpoglyphus* and *Tyrolichus.* In an even more particular embodiment, the first source of food comprises one of the following combinations of two populations of astigmatid mites: *Suidasia* and *Carpoglyphus; Suidasia* and *Tyrolichus; Carpoglyphus* and *Tyrolichus; Carpoglyphus* and *Tyreophagus; Suidasia* and *Tyreophagus; Carpoglyphus* and *Aleuroglyphus; Suidasia* and *Aleuroglyphus; Tyrolichus* and *Tyreophagus; Acarus* and *Tyrolichus; Acarus* and *Aleuroglyphus; Acarus* and *Tyreophagus.* In another embodiment, the first source of food comprises three populations of different astigmatid mites, more particularly, *Suidasia, Carpoglyphus* and *Tyrolichus,* or *Carpoglyphus, Suidasia* and *Tyreophagus, or Carpoglyphus, Tyrolichus* and *Tyreophagus, or Carpoglyphus, Acarus and Tyreophagus, or Carpoglyphus, Suidasia* and *Aleuroglyphus.*

In a particular embodiment, the first source of food is released in the plant or crop at a plurality of 'spots'. The density of spots of the first source of food in the crop is preferably between 1000 spots /m² and 1 spot/25 m². More preferably, the density of spots of first source of food is between 3 spots /m² and 1 spot/5 m², most preferably between 1 spot/m² and 1 spot/2 m². However, even higher densities of spots of first source of food in the crop are possible, especially when the material is sprayed by machinery.

In a preferred embodiment, the astigmatid mites are sprayed onto the crop. In this embodiment, a vast number of small spots of astigmatid mites are applied to the crop. Depending on the method of application these spots of astigmatid mites supply can be located on leaves and other parts of a plant such as stipes, stalks, flowers, etc. These spots can also be located on the ground.

In a particular embodiment, the density of the individuals of the mite species belonging to the Astigmata in the first sources of food is between 100.000 and 50 million individuals/litre. Using a density in this range permits the application of a suitable number of individuals per spot on the plant. In a further preferred embodiment, the density of the individuals of the mite species belonging to the Astigmata is between 500.000 and 30 million individuals/litre, most preferably between 1 million and 20 million individuals/litre. At these densities, it is easy to apply a suitable number of individuals to each spot on the plant using known techniques. Independent of the density, in a preferred embodiment of the invention, when a carrier substrate is used, a volume between 0.01 ml and 50 ml of the first sources of food is placed on one spot. Preferably between 0.1 ml and 10 ml, most preferably between 0.3 ml and 2 ml of the first sources of food is placed on the same spot. These volumes can be located and applied on the plant by known techniques. They include known apparatus such as automatic or manually operated sprayers, syringes, dropping devices or spoons.

Although predator populations are commonly transported from the mass-rearing facilities to the crop at low temperatures, so that the activity of the insects is minimized, these predator insects can be supplied with food. Therefore, in a particular embodiment, the breeding population of the predator insect can be provided to the plant or crop together with a second source of food. This second source of food can comprise a natural food, for example pollen, prey arthropods, for example aphids, or a factitious prey or artificial foods, and mixtures thereof. When natural prey arthropods are used, species that are not pests of the target crop are selected. For instance, aphids from cereals that cannot survive and damage a horticultural or an orchard crop, when the last are the target crops, are selected. Alternatively, dead pest species are selected, like frozen dipteran larvae of *Ceratitis capitata.* Another preferred food are factitious prey, which are arthropods that are not naturally occurring in the living habitat of the predators. Preferably, pest insects of storage food, like eggs of lepidoptera moths, crustacean cysts of *Artemia* and nematodes. Most preferred factitious prey are the eggs of the moths *Ephestia kuehniella* and *Sitotroga cerealella.* The term "artificial food", when applied to insects, is defined by several authors as any diet that is not the natural food of the insect. However, the term 'artificial food' as used herein excludes the factitious prey. Then, this name encompasses all the various terms such as synthetic, chemically defined, purified, holidic, meridic, and oligidic that are used to describe insect diets, excluding the factitious prey or hosts. Examples are meat paste, cooked chicken eggs, yeast, sugar, water, vegetable oils, vitamins or aminoacids, with or without antimicrobial compounds (Carson Cohen, Insect Diets: Science and Technology (Second Edition) 2015, 439 pp. CRC Press, Boca Raton. ISBN 978-1-4665-9194-3). Therefore, in a particular embodiment, the artificial food is not a factitious prey.

Optionally, artificial foods containing sugars, fatty acids, vitamins and/or aminoacids, combined with or without preservatives, can also be supplied with the predator populations. In a particular embodiment, second source of food is selected from the group consisting of eggs of *Ephestia kuehniella,* cysts of *Artemia,* eggs of *Sitotroga cerealella,* pollen, nematodes, larvae of *Ceratitis capitata,* larvae and/or adults of *Drosophila melanogaster,* artificial foods, and a combination thereof.

The term "eggs of *Ephestia kuehniella",* as used herein, refers to the eggs of this moth, identified in the NCBI database by the Taxonomy ID: 40079.

The term "cysts of *Artemia",* as used herein, refers to the encapsulated eggs of the brine shrimp of the genus *Artemia,* identified in the NCBI database by the Taxonomy ID: 6660. The cyst of *Artemia* can be of any species of the genus.

The term "eggs of *Sitotroga cerealella",* as used herein, refers to refers to the eggs of this moth commonly known as Angoumois grain moth, and identified in the NCBI database by the Taxonomy ID: 347735.

The term "pollen", as used herein, refers to the fine to coarse powder containing the microgametophytes of seed plants. The pollen can be obtained from any seed plants, including *Typha, Ricinnus,* corn, *Pennisetum, Crotalaria,* almond, apple, plum, maize, date palm, maple and *Quercus.* Pollen can also be a mixture of species when it is bee-collected pollen.

The term "nematode", as used herein, refers to a phylum of pluricellular pseudocoelomate organisms of the group Ecdysozoa. Illustrative non-limitative examples of nematodes that can be used as source of food for the predator insects include *Panagrellus* and *Panagrolaimus.* The term "larvae of *Ceratitis capitata"* as used herein, refers to the larva stage of this insect commonly known as Mediterranean fruit fly or medfly, and identified in the NCBI database by the Taxonomy ID: 7213.

The term "larvae or adults of *Drosophila melanogaster*", as used herein, refers to the larva or adult stage of this fly, and identified in the NCBI database by the Taxonomy ID: 7227.

Steps (a) and (b) of the first method of the invention can be performed simultaneously or separately in any order, that is, the plant or crop can be first provided with the breeding population of the predator insect from the genus *Orius* and, after that, the plant can be provided with the first source of food for the predator insect comprising the population of an astigmatid mite; or the plant or crop can be first provided with the first source of food for the predator insect comprising the population or an astigmatid mite and, after that, the plant or crop can be provided with the breeding population of the predator insect from genus *Orius;* or the plant or crop can be provided with the breeding population of the predator insect from the genus *Orius* and with the first source of food for the predator insect comprising the population or an astigmatid mite simultaneously.

In a particular embodiment, when the population of the predator insect and the first source of food comprising the population of an astigmatid mite are provided to the plant or crop simultaneously, they can be provided inside a common housing, for example, both populations can be spread by hand into cardboard cages that are hang on the plants.

The fist method of the invention comprises allowing the predator insect to prey on the astigmatid mite. In case the population of the predator insect and the population of the astigmatid mite are provided to the crop simultaneously with any kind of physical separation, this means allowing both populations to be in physical contact.

In a particular embodiment, the first method of the invention comprises providing the plant or crop with one or more of the following combinations of predator insects and astigmatid mites:
- *Orius laevigatus* as predator and *Acarus siro* as astigmatid mite.
- *Orius laevigatus* as predator and *Aleuroglyphus ovatus* as astigmatid mite.
- *Orius laevigatus* as predator and *Suidasia medanensis* as astigmatid mite.
- *Orius laevigatus* as predator and *Tyrolichus casei* as astigmatid mite.
- *Orius laevigatus* as predator and *Thyreophagus entomophagus* as astigmatid mite.
- *Orius laevigatus* as predator and *Carpoglyphus lactis* as astigmatid mite.
- *Orius insidiosus* as predator and *Acarus siro* as astigmatid mite.
- *Orius insidiosus* as predator and *Aleuroglyphus ovatus* as astigmatid mite.
- *Orius insidiosus* as predator and *Suidasia medanensis* as astigmatid mite.
- *Orius insidiosus* as predator and *Tyrolichus casei* as astigmatid mite.
- *Orius insidiosus* as predator and *Thyreophagus entomophagus* as astigmatid mite.
- *Orius insidiosus* as predator and *Carpoglyphus lactis* as astigmatid mite.

### Method for controlling a pest in a crop

In a second aspect, the invention relates to a method for controlling a pest in a crop comprising:
(a) providing the crop with at least a breeding population of an insect from the genus *Orius,* wherein said insect is a predator for said pest and
(b) providing the crop with a first source of food for the predator insect, wherein said first source of food comprises a population of an astigmatid mite,
wherein the astigmatid mite is a storage mite from a family selected from the group consisting of Acaridae, Carpoglyphidae, Glycyphagidae and Suidasiidae.

The term "pest", as used herein, refers to plant pest, and is understood to include any species, strain or biotype of plant, animal or pathogenic agent injurious for plant or plant products. The pests that can be controlled by the second method of the invention are animals that are prey for the predator insects from the genus *Orius.* In a particular embodiment, the pest is selected from the group consisting of thrips, whiteflies, aphids and moths.

The term "thrips", as used herein, includes any member of the order Thysanoptera. The order Thysanoptera includes the suborders Terebrantia and Tubulifera, the super families of Aeolothripoidea, Thripoidea, and Merothripoidea, and the families of Aeolothripidae, Heterothripidae, Thripidae, Uzelothripidae, and Phlaeothripidae. Specific varieties of thrips include greenhouse thrips ( *Heliothrips haemorrhoidalis*), banded greenhouse thrips (*Hercinothrips femoralis*), flower thrips (*Frankliniella tritici*), Western flower thrips (WFT) (*Frankliniella occidentalis*), onion or tobacco thrips (*Thrips tabaci*), citrus thrips (*Scirtothrips aurantii* and *Scirtothrips citri*), cereals thrips (*Limothrips cerealium*), pea thrips (*Kakothrips robustus*), lily bulb thrips (Liothrips), black hunter thrips (*Leptothrips mali*), coffee thrips (Diarthrothrips), avocado thrips (*Scirtothrips perseae*), Thrips palmi, fruit tree thrips (*Taeniothrips inconsequens*), gladiolus thrips (*Taeniothrips simplex*), azalea thrips (*heterothrips azaleae*), olive thrips (*Liothrips oleae*), six-spotted thrips (*Scolothrips sexmaculatus*), and cotton thrips (caliothrips sp. and Frankliniella sp). In a particular embodiment, the thrips is from a genus selected from the group consisting of *Frankliniella, Thrips* and *Hoplandrothrips.* In a more particular embodiment, the thrips is from a species selected from the group consisting of *Frankliniella occidentalis, Thrips tabaci, T. palmi, T. simplex, T. fuscipennis, T. angusticeps and Heliothrips haemorrhoidalis.*

The term "whiteflies", as used herein, refers to insects of the order Hemiptera that typically feed on the undersides of plant leaves. Whiteflies comprise the family Aleyrodidae, the only family in the superfamily Aleyrodoidea. Main pest species of whiteflies include *Aleurocanthus woglumi* (citrus blackfly), *Aleyrodes proletella* (cabbage whitefly), *Bemisia tabaci* (silverleaf whitefly), *Trialeurodes vaporariourum* (greenhouse whitefly).

The term "aphids", as used herein, refers to plant pest insects belonging to the family Aphididae, including but not limited to *Aphis gossypii, A. fabae, A. glycines, A. nerii, A. nasturtii, Myzus persicae, M. cerasi, M. ornatus, Nasonovia* especially *N. ribisnigri, Macrosiphum* especially *M. euphorbiae, Aulacorthum* especially *A. solani* and *Brevicoryne.*

The term "moth", as used herein, refers to a group of insects that includes all member of the order Lepidoptera that are not butterflies. The caterpillar of moths feed on leaves or other parts of plants and therefore, moths can be considered as plant pests. Illustrative non-limitative example of moths that can be controlled with the second method of the invention include tomato leafminer (*Tuta absoluta*), beet armyworm (Spodoptera exigua), (Spodoptera littoralis), cotton bollworm (*Helicoverpa armigera*), tomato Looper (*Chrysodeixis chalcites*), codling moth (*Cydia pomonella*), holm oak leaf-mining moths (*Phlyllonoricter messaniella* and *Ectoedemia heringella*), horse chestnut leaf-mining moth (*Cameriaria ohridella*), leek moth (*Acrolepiopsis assectella*), pea moth (*Cydia nigricana*), plum moth (*Grapholita funebrana*), winter moth (*Operophtera brumata, Erannis defoliaria, Alsophila aescularia*) and tortrix moth (*Cacoecimorpha pronubana* and *Epiphyas postvittana*)*.* In a particular embodiment the moth is *Tuta absoluta.*

All the other terms have been previously defined in connection with the first method of the invention. The definitions and particular and preferred embodiments of said terms also apply to the second method of the invention.

### Uses and compositions

In another aspect, the invention relates to the use of an astigmatid mite for rearing a predator insect for the genus *Orius* on a crop, wherein said astigmatid mite is not *Tyrophagus putrescentiae,* and wherein said astigmatid mite is a storage mite from a family selected from the group consisting of Acaridae, Carpoglyphidae, Glycyphagidae and Suidasiidae.

All the terms have been previously defined in connection with the first method of the invention. The definitions and particular and preferred embodiments of said terms also apply to the use of the invention.

In another aspect, the invention relates to a composition comprising at least a breeding population of a predator insect from the genus *Orius* and a first source of food for said predator insect, wherein said first source of food comprises a population of an astigmatid mite, wherein said population of an astigmatid mite is not a population of *Tyrophagus putrescentiae* or a population of *Carpoglyphus lactis,* wherein the breeding population of the predator insect and the first source of food are physically separated so that there is no contact between the population of the predator insect and the population of the astigmatid mite, and wherein the astigmatid mite is an storage mite from a family selected from the group consisting of Acaridae, Carpoglyphidae, Glycyphagidae and Suidasiidae.

The term "composition", as used herein, refers to a combination of compounds or ingredients, in this particular case, the breeding population of the predator insect and the first source of food.

In a particular embodiment, the astigmatid mite is not *Tyrophagus putrescentiae.* In a particular embodiment, the astigmatid mite is not *Carpoglyphus lactis.* In a particular embodiment, the astigmatid mite is not *Tyrophagus putrescentiae* nor *Carpoglyphus lactis.* In a particular embodiment, the predator anthocorid is not *Orius laevigatus.* In a particular embodiment, the composition does not comprise *Tyrophagus putrescentiae* and *Orius laevigatus.* In a particular embodiment, the composition does not comprise *Tyrophagus putrescentiae* and *Nesidiocoris tenuis.* In a particular embodiment, the composition does not comprise *Tyrophagus putrescentiae* and *Macrolophus pygmaeus.* In a particular embodiment, the composition does not comprise *Carpoglyphus lactis* and *Orius laevigatus.* In a particular embodiment, the composition does not comprise *Carpoglyphus lactis* and *Nesidiocoris tenuis.* In a particular embodiment, the composition does not comprise *Carpoglyphus lactis* and *Macrolophus pygmaeus.* In a particular embodiment, the composition does not comprise *Tyrophagus putrescentiae* and *Orius laevigatus,* or *T. putrescentiae* and *Nesidiocoris tenuis,* or *T. putrescentiae* and *Macrolophus pygmaeus,* or *Carpoglyphus lactis* and *Orius laevigatus,* or *Carpoglyphus lactis* and *Nesidiocoris tenuis,* or *Carpoglyphus lactis* and *Macrolophus pygmaeus.*

In a particular embodiment, the composition comprises one the following combinations of predator and prey:
- *Orius laevigatus* as predator and *Acarus siro* as astigmatid mite.
- *Orius laevigatus* as predator and *Aleuroglyphus ovatus* as astigmatid mite.
- *Orius laevigatus* as predator and *Suidasia medanensis* as astigmatid mite.
- *Orius laevigatus* as predator and *Tyrolichus casei* as astigmatid mite.
- *Orius laevigatus* as predator and *Thyreophagus entomophagus* as astigmatid mite.
- *Orius insidiosus* as predator and *Acarus siro* as astigmatid mite.
- *Orius insidiosus* as predator and *Aleuroglyphus ovatus* as astigmatid mite.
- *Orius insidiosus* as predator and *Suidasia medanensis* as astigmatid mite.
- *Orius insidiosus* as predator and *Tyrolichus casei* as astigmatid mite.
- *Orius insidiosus* as predator and *Thyreophagus entomophagus* as astigmatid mite.

In a particular embodiment, the composition further comprises a second source of food for the predator insect.

The term "second source of food" has been previously defined in connection with the first method of the invention. All the particular and preferred embodiments of this term fully apply to the composition of the invention. In a particular embodiment, the wherein the second source of food is selected from the group consisting of eggs of *Ephestia kuehniella,* cysts of *Artemia,* eggs of *Sitotroga cerealella,* pollen, nematodes, larvae of *Ceratitis capitata,* larvae and/or adults of *Drosophila melanogaster,* artificial foods, and a combination thereof.

In another aspect, the invention refers to the use of the composition of the invention for controlling a pest in a plant, wherein the pest is a prey for the predatory insect.

All the terms have been previously defined in connection with the first and second method of the invention. The definitions and particular and preferred embodiments of said terms also apply to the use of the composition of the invention.

### Methods for obtaining a strain with increased tolerance to a diet based on a factitious food (not encompassed by the claimed invention)

In another aspect, it is disclosed a method of obtaining a strain of a predator insect from the family Miridae or from the family Anthocoridae with an increased tolerance to a diet based on a factitious food, hereinafter third disclosed method, the method comprising artificially selecting a strain of the predator insect for its tolerance to a diet based on pollen under a selective pressure, wherein said selective pressure comprises using pollen as the main source of food. The terms "predator insect", "family Miridae", "family Anthocoridae" have been previously defined in connection with the first method of the invention. All the particular and preferred embodiments of said terms defined for the first method of the invention also apply to the third disclosed method.

In a particular aspect not encompassed by the claimed invention, the predator insect from the family Miridae is from a genus selected from the group consisting of *Macrolophus, Nesidiocoris, Dicyphus, Deraeocoris*, *Engytatus, Tupiocoris, Campyloneuropsis, Cyrtopeltis, Pilophorus, Campylomma, Cyrtorhinus, Orthotylus, Eurotas.* In a more particular aspect not encompassed by the claimed invention, the predator insect from the family Miridae is from a species selected from the group consisting of *Macrolophus pygmaeus, M. costalis, M. basicornis, Nesidiocoris tenuis, N. volucer, N. callani, Dicyphus bolivari., D. errans, D. hesperus, D. marrocanus, D. geniculatus, D. tamaninii, Engytatus varians, E. modestus, Tupiocoris cucurbitaceus, Campyloneuropsis infumatus, Cyrtopeltis callosus, Deraeocoris brevis, D. nebulosus, Pilophorus typicus, P. gallicus, Campylomma verbasci, C. chinensis, Cyrtorhinus lividipennis, Orthotylus marginalis* and *Eurotas brasilianus.*

In a particular aspect not encompassed by the claimed invention, the predator insect from the family Miridae is from the genus *Nesidiocoris* or from the genus *Dicyphus* or from the genus *Macrolophus,* more particularly from the species *Nesidiocoris tenuis* or from the species *Dicyphus bolivari* or from the species *Macrolophus pygmaeus* or from the species *Dicyphus hesperus.*

In a particular aspect not encompassed by the claimed invention, the predator insect from the family Anthocoridae is an insect from a genus selected from the group consisting of *Orius, Anthocoris, Blaptostethus, Montandoniola* and *Xylocoris.*

In a particular aspect not encompassed by the claimed invention, the predator insect from the family Anthocoridae is an insect from a species selected from the group consisting of *Orius laevigatus, O. insidiosus, O. majusculus, O. niger, O. albidipennis, O. minutus, O. thripoborus, O. naivashae, O. strigicollis, O. sauteri, O. tristicolor, O. nagaii, O. antillus, O. limbatus, O. thripoborus,* O. *naiashae, O. horvathi, O. vicinus, O. pumilio, Orius laticollis, O. lindbergi, Anthocoris nemorum, A. nemoralis, A. confusus* and A. *minki, Blaptostethus pallescens, Montandoniola confuse, M. pictipennis* and *Xylocoris flavipes.*

In a more particular aspect not encompassed by the claimed invention, the predator insect from the family Anthocoridae is *Orius laevigatus.*

The term "diet based on a factitious food" means that the factitious food represents the main source of calories of the diet. In a particular aspect not encompassed by the claimed invention, at least 60%, at least 70%, at least 80%, at least 90%, at least 95% or the 100% of the calories of the diet are provided by the factitious food. The term "factitious food", as used herein, refers to any food different to the one that the animal, in this case the predator insect from the families Miridae or Anthocoridae, eats on its natural habitat. In a particular aspect not encompassed by the claimed invention, the factitious food comprises a factitious prey, an artificial food or a mixture thereof. In a particular aspect not encompassed by the claimed invention, the factitious food is not pollen.

The term "factitious prey", as used herein, refers to prey that serve as food for predators although they are not the natural prey for the predator. In a particular aspect not encompassed by the claimed invention, the factitious prey is selected from the group consisting of an astigmatid mite, eggs of *Ephestia kuehniella,* cysts of *Artemia,* eggs of *Sitotroga cerealella,* nematodes, larvae of *Ceratitis capitate* and larvae and/or adults of *Drosophila melanogaster.* All these terms have been previously defined in connection with the first method of the invention.

In a particular aspect not encompassed by the claimed invention, the factitious prey is an astigmatid mite.

In a particular aspect not encompassed by the claimed invention, the astigmatid mite is a storage food mite. In a particular aspect not encompassed by the claimed invention, the storage food mite is from a family selected from the group consisting of Acaridae, Carpoglyphidae, Glycyphagidae, Pyroglyphidae, Chortoglyphagidae and Suidasiidae.

In a more particular aspect not encompassed by the claimed invention, the storage food mite is from a genus selected from the group consisting of *Acarus, Tyrophagus, Aleuroglyphus, Lardoglyphus, Caloglyphus, Suidasia, Thyreophagus, Carpoglyphus, Glycyphagus, Tyrolichus, Lepidoglyphus, Blomia* and *Chortoglyphus.* In an even more particular aspect not encompassed by the claimed invention, the storage food mite is from a species selected from the group consisting of *Suidasia medanensis, Suidasia nesbitti, Lepidoglyphus (Glycyphagus) destructor, Blomia freeman, Carpoglyphus lactis, Carpoglyphus munroi, Carpoglyphus wardleorum, Lardoglyphus konoi, Aleuroglyphus ovatus, Tyrolichus casei, Thyreophagus entomophagus, Chortoglyphus arcuatus* and *Acarus siro.*

In a particular aspect not encompassed by the claimed invention, the storage food mite is from a family selected from the group consisting of Acaridae, Carpoglyphidae and Suidasiidae, more particularly from a genus selected from the group consisting of *Acarus, Carpoglyphus, Suidasia, Tyreophagus, Aleuroglyphus* and *Tyrolichus,* even more particularly from a species selected from the group consisting of *Acarus siro, Carpoglyphus lactis, Suidasia medanensis, Tyreophagus entomophagus, Aleuroglyphus ovatus* and *Tyrolichus casei.*

The term "artificial food" has been previously defined.

The third disclosed method allows obtaining a strain of a predator insect from the families Miridae or Anthocoridae with an increased tolerance to a diet based on a factitious food, preferably, a factitious prey, more preferably an astigmatid mite.

The term "tolerance" as used herein, refers to the ability of the predator insect to achieve an efficient development, reaching the stage of sexually mature adult and being able of reproduce. The term "increased tolerance", as used herein, refers to an augmented tolerance compared to a predator insect from the same species that has not been selected under a selective pressure comprising using pollen as the main source of food. In a particular aspect not encompassed by the claimed invention, the term "increased tolerance" refers to an augmented tolerance compared to a wild predator insect from the same species. In another particular aspect not encompassed by the claimed invention, the term "increased tolerance" refers to an augmented tolerance compared to an insect from the same species that is acclimated to pollen, but that has not been selected for its tolerance to pollen. The term "acclimated" means than the insect derives from a population that has been feed with a suboptimal diet based on pollen, for example, providing pollen every 2-3 days and a suboptimal quantity of *Ephestia* eggs every week.

In a particular aspect not encompassed by the claimed invention, an increased tolerance means increased nymph survival, increased fecundity and increased longevity.

Therefore, in a particular aspect not encompassed by the claimed invention, the strain of the predator insect obtained by the third method of the invention has increased nymph survival compared to a predator insect of the same species that has not been selected for its tolerance to a diet based on a factitious food. The term "nymph survival" refers to the percentage of nymphs that reach adulthood of the total initial number of nymphs emerged from the eggs. The nymph survival is considered increased when it is at least a 1%, at least a 2%, at least a 3%, at least a 4%, at least a 5%, at least a 10%, at least a 20%, at least a 30%, at least a 40%, at least a 50%, at least a 60%, at least a 70%, at least a 80%, at least a 90%, at least a 95% higher or even higher than the nymph survival of a predator insect from the same species that has not been selected for its tolerance to a diet based on a factitious food.

In another particular aspect not encompassed by the claimed invention, the strain of the predator insect obtained by the third disclosed method has increased fecundity compared to a predator insect of the same species that has not been selected for its tolerance to a diet based on a factitious food. The term "fecundity" refers to the ability of the predator insect to lay eggs, being measured as the average number of eggs laid by a female. In a particular aspect not encompassed by the claimed invention, the fecundity is expressed as a rate of egg or offspring production or as a total number of eggs or offspring produced by the predator insect during a period of time. In a particular aspect not encompassed by the claimed invention, the fecundity is determined through a period of time of 10 days. In another particular aspect not encompassed by the claimed invention, the fecundity is "lifetime fecundity", that is, fecundity throughout the life of the individual. The fecundity is considered increased when it is at least a 1%, at least a 2%, at least a 3%, at least a 4%, at least a 5%, at least a 10%, at least a 20%, at least a 30%, at least a 40%, at least a 50%, at least a 60%, at least a 70%, at least a 80%, at least a 90%, at least a 95% higher or even higher than the fecundity of a predator insect from the same species that has not been selected for its tolerance to a diet based on a factitious food.

In another particular aspect not encompassed by the claimed invention, the strain of the predator insect obtained by the third disclosed method has increased longevity compared to a predator insect of the same species that has not been selected for its tolerance to a diet based on a factitious food. The term "longevity" refers to the lifespan of the predator insect as adult. The longevity is considered increased when it is at least a 1%, at least a 2%, at least a 3%, at least a 4%, at least a 5%, at least a 10%, at least a 20%, at least a 30%, at least a 40%, at least a 50%, at least a 60%, at least a 70%, at least a 80%, at least a 90%, at least a 95% higher or even higher than the longevity of a predator insect from the same species that has not been selected for its tolerance to a diet based on a factitious food.

The term "artificially selecting" means that the predator insect is growth under conditions of selective pressure introduced by the human being, in particular, the selective pressure comprises using pollen as the main source of food. The term "main source of food", as used herein, means that pollen is the main source of calories of the diet. In a particular aspect not encompassed by the claimed invention, at least 60%, at least 70%, at least 80%, at least 90%, at least 95% or the 100% of the calories of the diet are provided by pollen. In a particular aspect not encompassed by the claimed invention, selective pressure consists on providing pollen as the only source of food.

In a particular aspect not encompassed by the claimed invention, each selected population of the predator insect is maintained before the next selection step with a diet comprising mainly pollen and also other factitious foods, such as *Ephestia* eggs, before the next cycle of selection. For example, the selected population is bred with a diet comprising pollen and once a week, other factitious food, such as *Ephestia* eggs.

Artificial selection comprises breeding a population of the predator insect under conditions of selective pressure, in particular, using pollen as the main source of food, and selecting those individuals that better perform under said conditions. The individuals that better performed can be selected based on any of the parameters "nymph survival", "fecundity" or "longevity", but are preferably selected based on the parameter "fecundity". For making this selection, the individuals, preferably the female individuals having a nymph survival, fecundity and/or longevity, higher than the mean of any of those parameters in the whole population are selected.

In a particular aspect not encompassed by the claimed invention, the offspring from the individuals previously selected are selected and mixed so that they form a new population. With this new population, the steps of breeding the population under the selective pressure comprising using pollen as the main source of food, selecting the individuals, preferably the female individuals, with a better performance, preferably with a higher fecundity, selecting the offspring from these individuals and mixing the selected offspring to form a new population is repeated at least once, at least twice, at least 3 times, at least 4 times, at least 5 times, at least 6 times, at least 7 times, at least 8 times, at least 9 times, at least 10 times at least 11 times, at least 15 times, at least 20 times, at least 30 times, at least 40 times, at least 50 times or more. Preferably the whole selection cycles are performed at least 11 times.

In a particular aspect not encompassed by the claimed invention, the third disclosed method comprises:
(i) breeding a population of the predator insect, preferably *Orius laevigatus,* using pollen as the main source of food,
(ii) selecting the female individuals from the population used in step (i) that have a higher fecundity,
(iii) selecting the offspring from the female individuals selected in step (ii),
(iv) mixing the offspring selected in step (iii) to form a new population and
(v) repeating steps (i) through (iv) at least once, preferably at least 5 times, more preferably at least 10 times.

In another aspect not encompassed by the claimed invention, it is disclosed a method of obtaining a strain of a predator insect from the family Miridae or from the family Anthocoridae with an increased tolerance to a diet based on a factitious food, the method comprising artificially selecting a strain of the predator insect for its tolerance to low temperatures under a selective pressure, wherein said selective pressure comprises rearing the population of the predator insect at a temperature lower than 20°C.

All the terms previously defined for the method of obtaining a strain of a predator insect with increased tolerace to a diet based on a factitious food based using a selective pressure comprising feeding the insect with pollen fully apply to the method that uses cold as the selective pressure.

The term "low temperature", as used herein, refers to a temperature lower than 20°C, for example, 19°C, 18°C, 17°C, 16°C, 15°C, 14°C or lower, preferably 15°C. The selective pressure comprises rearing the population of the predator insect at a temperature lower than 20°C, preferably a temperature higher than 9°C, for example, 19°C, 18°C, 17°C, 16°C, 15°C, 14°C, 13°C, 12°C, 11°C or 10°C.

In a particular aspect not encompassed by the claimed invention, the third disclosed method comprises:
(i) breeding the population of the predator insect, preferably *Orius laevigatus* at a temperature lower than 20°C,
(ii) selecting the female individuals from the population used in step (i) that have a higher fecundity,
(iii) selecting the offspring from the female individuals selected in step (ii),
(iv) mixing the offspring selected in step (iii) to form a new population and
(vi) repeating steps (i) through (iv) at least once, preferably at least 5 times, more preferably at least 10 times.

### Strain of a predator insect with increased tolerance to a diet based on a factitious food (not encompassed by the claimed invention)

In another aspect not encompassed by the claimed invention, it is disclosed a strain of a predator insect obtainable by the methods for obtaining a strain with increased tolerance to a diet based on a factitious food.

In a particular aspect not encompassed by the claimed invention, the predator insect from the family Miridae is from the genus *Nesidiocoris, Dicyphus* or *Macrolophus,* more particularly from the species *Nesidiocoris tenuis, Macrolophus pygmaeus, Dicyphus bolivari, Dicyphus hesperus,* or *Dicyphus errans.*

In a particular aspect not encompassed by the claimed invention, the predator insect from the family Anthocoridae is an insect from a genus selected from the group consisting of *Orius, Anthocoris, Blaptostethus, Montandoniola* and *Xylocoris.*

In a particular aspect not encompassed by the claimed invention, the predator insect from the family Anthocoridae is an insect from a species selected from the group consisting of *Orius laevigatus, O. insidiosus, O. majusculus, O. niger, O. albidipennis, O. minutus, O. thripoborus, O. naivashae, O. strigicollis, O. sauteri, O. tristicolor, O. nagaii, O. antillus, O. limbatus, O. thripoborus,* O. *naiashae, O. horvathi, O. vicinus, O. pumilio, Orius laticollis, O. lindbergi, Anthocoris nemorum, A. nemoralis, A. confusus* and A. *minki, Blaptostethus pallescens, Montandoniola confuse, M. pictipennis* and *Xylocoris flavipes.*

In a more particular aspect not encompassed by the claimed invention, the predator insect from the family Anthocoridae is *Orius laevigatus.*

As shown in Example 13, when the predator insect is *Orius laevigatus,* the strain of a predator insect obtainable by the methods for obtaining a strain with increased tolerance to a diet based on a factitious food is characterized by a particular amplification pattern when the genome of the strain is amplified by PCR with primers for detecting the SSR (single sequence repetition) Rp02. Therefore, in a particular aspect not encompassed by the claimed invention, when the predator insect is *Orius laevigatus,* the strain of a predator insect obtainable by the methods for obtaining a strain with increased tolerance to a diet based on a factitious food is characterized in that when the genome of the strain is amplified by PCR using the pair or primers of SEQ ID NO: 1 and SEQ ID NO: 2 the following fragments are obtained in at least 70% of the individuals of the strain:
(a) a fragment of 780-840 base pairs or
(b) a fragment of 1280-1360 base pairs, a fragment of 580-640 base pairs and a fragment of 420-480 base pairs.
Particular aspect not encompassed by the claimed invention of said fragments have been previously defined. In particular, the strain of the predator insect from the species *Orius laevigatus* obtained by the method based on the selective preasure comprising a diet based on pollen is characterized in that when the genome of the strain is amplified by PCR using the pair of primers of SEQ ID NO: 1 and SEQ ID NO: 2 a fragment of 780-840 bp is obtained in at least 70%, at least 75%, at least 80%, at least 85%, at least 90%, at least 95%, at least 98% or the 100% of the individuals of the strain of *Orius laevigatus.*

Similarly, the strain of the predator insect from the species *Orius laevigatus* obtained by the method based on the selective pressure based on low temperature is characterized in that when the genome of the strain is amplified by PCR using the pair of primers of SEQ ID NO: 1 and SEQ ID NO: 2 fragment of 1280-1360 base pairs, a fragment of 580-640 base pairs and a fragment of 420-480 base pairs are obtained in at least 70%, at least 75%, at least 80%, at least 85%, at least 90%, at least 95%, at least 98% or the 100% of the individuals of the strain of *Orius laevigatus.*

The term "PCR" or "polymerase chain reaction", as used herein, relates to a DNA amplification process by means of polymerase chain reaction, that allows amplifying a single or a few copies of a DNA sequence across several orders of magnitude, generating as a result thousand to millions of copies of a particular DNA sequence. The PCR method relies on thermal cycling, consisting of cycles of repeated heating and cooling of the reaction for DNA melting and enzymatic replication of the DNA. Primers (short DNA fragments) containing sequences complementary to the target region along with a DNA polymerase (after which the method is named) are key components to enable selective and repeated amplification. As PCR progresses, the DNA generated is itself used as a template for replication, setting in motion a chain reaction in which the DNA template is exponentially amplified. PCR can be extensively modified to perform a wide array of genetic manipulations. The PCR process is well known in the art and is thus not described in detail herein.

In a particular aspect not encompassed by the claimed invention, the PCR is performed using the program shown in Figure 10.

In a more particular aspect not encompassed by the claimed invention, the PCR is performed under the following conditions: using 1 unit of VWR^{®} Red Taq DNA Polymerase, 1X Key Buffer, 3mM Cl₂Mg, 0.5mM of DNTPs, 10pM from each primer, and 5-25ng genomic DNA per reaction, and when the PCR is run in an Eppendorf Mastercycler Pro according to the program shown in Figure 10. Results can be analyzed by electrophoresis in 1.2% agarose gel.

In another aspect not encompassed by the claimed invention, it is disclosed a strain of a predator insect of the species *Orius laevigatus* with increased tolerance to a diet based on a factitious food, wherein said strain is characterized in that when the genome of the strain is amplified by PCR using the pair or primers of SEQ ID NO: 1 and SEQ ID NO: 2 the following fragments are obtained:
(a) a fragment of 780-840 base pairs or
(b) a fragment of 1280-1360 base pairs, a fragment of 580-640 base pairs and a fragment of 420-480 base pairs.

### Method for controlling a pest in a crop with a strain with increased tolerance to a factitious food

In another aspect, the invention relates to a method for controlling a pest in a crop comprising providing the crop with at least a breeding population of a predator insect from the genus *Orius,* wherein the predator insect is from a strain with increased tolerance to a diet based on a factitious food, wherein said strain is obtained by
(a) a method comprising artificially selecting a population of the predator insect for its tolerance to a diet based on pollen under a selective pressure, wherein said selective pressure comprises using pollen as the main source of food or
(b) a method comprising artificially selecting a population of the predator insect for its tolerance to low temperatures under a selective pressure, wherein said selective pressure comprises rearing the population of the predator insect at a temperature lower than 20°C.

The methods (a) and (b) are the methods for for obtaining a strain with increased tolerance to a diet based on a factitious food previously defined.

In a seventh aspect, the invention relates to the use of a predator insect from the genus *Orius,* for controlling a pest in a crop, wherein the predator insect is from a strain with increased tolerance to a diet based on a factitious food, wherein said strain is obtained by
(a) a method comprising artificially selecting a population of the predator insect for its tolerance to a diet based on pollen under a selective pressure, wherein said selective pressure comprises using pollen as the main source of food, and wherein the pest is a prey for the predator insect or
(b) a method comprising artificially selecting a population of the predator insect for its tolerance to low temperatures under a selective pressure, wherein said selective pressure comprises rearing the population of the predator insect at a temperature lower than 20°C.

The methods for obtaining the strain with increased tolerance to a diet based on a factitious food is the method previously defined.

All the terms of the method of the sixth aspect and of the use of the seventh aspect have been previously defined. The particular and preferred embodiments of these terms regarding the other aspects of the invention also apply to the sixth and seventh aspects.

In a particular embodiment, the method further comprises providing the crop with a first source of food for the predator insect, wherein said first source of food is a factitious food. In a more particular embodiment, said factitious food is selected from the group consisting of a factitious prey, an artificial food or a mixture thereof. In an even more particular embodiment, the factitious prey is selected from the group consisting of an astigmatid mite, eggs of *Ephestia kuehniella,* cysts of *Artemia,* eggs of *Sitotroga cerealella,* nematodes, larvae of *Ceratitis capitate* and larvae and adults of *Drosophila melanogaster.*

In a particular embodiment, the crop is selected from the group consisting of vegetables, orchards, vineyard, olive trees, berries and ornamental plants, including pot plants and cut flowers.

In a particular embodiment, the pest is selected from the group consisting of thrips, whiteflies, aphids and moths. In a more particular embodiment, the thrips is from a genus selected from the group consisting of *Frankliniella, Thrips* and *Hoplandrothrips.* In an even more particular embodiment, the thrips is from a species selected from the group consisting of *Frankliniella occidentalis, Thrips tabaci, T. palmi, T. simplex, T. fuscipennis, T. angusticeps and Heliothrips haemorrhoidalis.*

In another aspect, the invention relates to a method for controlling a pest in a crop comprising providing the crop with at least a breeding population of a predator insect from the species *Orius laevigatus,* wherein the predator insect is from a strain with increased tolerance to a diet based on a factitious food, wherein said strain is characterized in that when the genome of the strain is amplified by PCR using the pair or primers of SEQ ID NO: 1 and SEQ ID NO: 2 the following fragments are obtained in at least 70% of the individuals of the strain:
(a) a fragment of 780-840 base pairs or
(b) a fragment of 1280-1360 base pairs, a fragment of 580-640 base pairs and a fragment of 420-480 base pairs.

In a particular embodiment, the strain of the predator insect is obtained by the methods for obtaining a strain of a predator insect with increased tolerance to a diet based on a factitious food of the invention previously described.

The invention will be described by way of the following examples which are to be considered as merely illustrative and not limitative of the scope of the invention.

### EXAMPLES

### Example 1. Introduction of astigmatid mites as food for Orius laevigatus in pepper crop

### Objectives

To evaluate the introduction of astigmatid mites as in-crop food, when used mixed or not with additional high-quality described prey, to improve the establishment of *Orius laevigatus* (Heteroptera: Anthocoridae) in a seedless variety of pepper. More specifically, to evaluate the establishment of this anthocorid in a pepper variety with low amount and/or poor quality of pollen, where the predator was not able to be established in previous years despite releasing high dosages, due to this lack of pollen.

### Materials & method

The trial was conducted in a commercial greenhouse in the south of Spain (Almeria) in the spring-summer of 2019. The greenhouse was a typical 'parral' structure used in the Mediterranean area with 2,400 m². A pepper crop of a mini Capia type variety Pepperito was transplanted in May the 10^{th}. Two introductions of O. *laevigatus* were performed, one with a dosage of 5 individuals/m² in June the 21^{st}, and another one with 3 individuals/m², in July the 5^{th}. Four treatments were evaluated applying different in-crop factitious prey for the predator:
- (1) *Carpoglyphus lactis* (Acari: Astigmata)
- (2) *C. lactis* complemented with cysts of the brine shrimp *Artemia* (Branchiopoda: Artemiidae)
- (3) *C. lactis* complemented with eggs of the Angoumois grain *moth Sitotroga cerealella* (Lepidoptera: Gelechiidae)
- (4) Control without food added

Treatments 1, 2 and 3 were applied in approximately 750 m², and treatment 4 (control) was applied in a surface of approximately 150 m². The grower did not want to establish a bigger area as a control plot, without a feeding system, since he had already released high amounts of *O. laevigatus* in two previous crops (same variety of peppers) without success. This control plot was separated with a plastic screen 2.5 m high to avoid movement of predators from/to the area where foods were introduced.

Populations of the astigmatid mite *C. lactis* produced and commercially sold by Agrobio (Powermite), which are packaged with bran and vermiculite as a substrate in cardboard bottles of 1 liter or paper bags of 5 liters (density between 1-5 million mites per liter), were introduced in treatments 1, 2 and 3, approximately every two weeks, in four dates: 21 of June, 5 and 19 of July, and 2 of August. The dosage introduced each feeding day in these 3 treatments was 37.5 liters/ha. To distribute the material a full spoon was released by hand (approximately 1.8 mL) in each plant.

Additionally, 10 grams of *Artemia* (250,000 cysts/gram) and 10 grams of *S. cerealella* (52,000 eggs/gram) were in introduced in each date in treatments 2 and 3 (approximately 135 grams per hectare), respectively. The cysts of brine shrimps and the eggs of the moth were homogeneously mixed with the populations of mites (about 3.6 g of *Artemia* or *S. cerealella* per liter of Powermite) and distributed together with the mites.

To evaluate the seasonal abundance of the populations of *O. laevigatus* and the main pests a weekly sampling was performed. The number of nymphs and adults of *O. laevigatus,* (on a basal, medium and higher position) and 2 flowers per plant from a total of 10 randomly selected plants per treatment. The three rows of plants in the borders between treatments were kept as a buffer area and were not considered when sampling.

Means and standard errors of predators, thrips and whiteflies were calculated per treatment and sampling date. The numbers of *O. laevigatus* were analyzed using an ANOVA per each sampling date and means separated using a Duncan test (SAS System 9.0).

### Results

Mean number (±SE) of nymphs and adults of *O. laevigatus* counted in the four treatments in each sampling date are represented in figure 1. The populations were well established in 100% of the plants in the 3 treatments with in-crop factitious prey added, while only 10-30% of the plants had predators in the control plot. The total mean number of individuals per two flowers was 1.26, 1.26, 1.20 and 0.26 in treatments 1, 2, 3 and 4, respectively, so it was about 5 times higher in the treatments with factitious prey compared to the control. The populations were significantly different among treatments on the dates: 19/07 (F= 7.4, p< 0.01), 26/07 (F=2.9, p=0.05), 2/08 (F= 4.2, p=0.01), 17/08 (F= 4.7, p< 0.01) and 22/08 (F= 3.1, P=0.03). Means were not different between treatments with factitious foods and significantly higher in all of them compared to the control treatment (with P=0.05).

### Conclusions

The introduction of the astigmatid mite *C. lactis* gave an unexpected result allowing the establishment of the populations of *O. laevigatus* in a variety of peppers type mini Capia. The populations of the predator remained at low numbers in the plants without added factitious prey, as expected. Similar results than in this control plot were counted in previous years, using the same high released dosages without added in-crop food, in several greenhouse pepper crops of the same variety. No successful establishment of *O. laevigatus* is achieved in this seedless variety, when no additional food is added, despite releasing up to 8 individuals/m², while no more than 2-3 individuals of *O. laevigatus*/m² are commonly released in other varieties of sweet pepper crops in the south of Spain. Most probably, the difficult development of the populations in the mini Capia are due to the poor quality of pollen, which is an alternative food for this polyphagous predator on peppers.

Importantly, thanks to the good establishment of the predator, the thrips populations were successfully controled for the first time in this variety of peppers. It is remarkable that the grower suffered important damages produced by thrips in the two previous years.

In this trial no differences were observed when the astigmatid mites were complemented with other factitious prey, compared to the use of only astigmatid mites. The eggs of *S. cerealella* and the cysts of *Artemia* are considered as high-quality foods, which are used or have been proposed as standard foods in the commercial mass-rearing of *O. laevigatus,* by the main biomanufacturers and several researchers. To date, astigmatids have been considered a low-quality food for *Orius laevigatus* and no other experiences have been described using these factitious mites as in-crop food, nor alone neither mixed with other factitious prey or foods. The improvement of the establishment of the populations of this anthocorid on a seedless variety of peppers with this affordable strategy offers a solution for this crop. This is also applicable to other crops without pollen or low quality of pollen, or crops where predators are released when plants have not yet developed the flowers, for example many ornamental crops like cut flowers and pot plants.

### Example 2. Introduction of astigmatid mites as food for Nesidiocoris tenuis in tomato crop (Comparative example, not being part of the invention)

### Objectives

To evaluate the introduction of astigmatid mites as in-crop food in order to improve the establishment of *Nesidiocoris tenuis* (Heteroptera: Miridae) in a greenhouse tomato crop. The strategy was compared to the standard procedure in the South of Spain, where commonly only one (occasionally two) additions of eggs of the moth *Ephestia kuehniella,* as a factitious prey, are introduced at the top of the plants, at the same time when the predators are released. In most of the cases the predators are released in the seedlings, before transplanting the crop, but sometimes, they are introduced during the first weeks after transplanting.

The strategy was also compared to the introduction of cysts of the brine shrimp *Artemia,* which is a factitious prey that has been proposed as an alternative in-crop food to the very expensive eggs of *E. kuehniella,* and it is already used in some tomato crops, mainly in high-tech glasshouses in Holland, U.K. and other north European countries.

### Materials & methods

The trial was conducted in a commercial greenhouse in the south of Spain (Almeria) in the autumn-spring of 2017/18. The plastic greenhouse had 4.500 m², with the typical 'parral' structure of the area, with a flat roof, with zenithal and lateral windows to offer ventilation, all covered with insect proof nets, and without active climate control, which are the structures comprising 80% Almeria's greenhouses. A tomato crop of the variety Genio (Clause) was transplanted on August the 23^{th}, 2017, with a density of 1.5 plants / m². The predator *N. tenuis* was released in the crop with a rate of 0.88 individuals/plant on September the 7th. The same day when predators were released a feeding was performed, following the standard procedure by the growers, introducing 10 g of eggs of the moth *E. kuehniella* every 1,000 predators.

Afterwards, three treatments were evaluated based on a regular introduction at the top of the plants of different in-crop foods:
- (1) *Suidasia medanensis* (Acari: Astigmata)
- (2) Cysts of the brine shrimp *Artemia* (Branchiopoda: Artemiidae)
- (3) Control without additional releases of in-crop food

Treatments 1 and 2 were applied in approximately 600 m² and were separated by the central corridor of the greenhouse. Treatment 3 (control) was applied in a surface of aprox. 1.300 m². An insect proof net was established as a barrier to separate physically the control treatment. The height of the barrier was about 2.5 meters, and it was considered that it was enough to minimize flying adults from/to the area where foods were introduced.

The populations of the mite *S. medanensis* were produced and packaged in the facilities of Agrobio, with bran and vermiculite as a substrate. It is a product commercially sold by Agrobio with the trade name Powerfood, packaged in cardboard bottles of 1 liter or paper bags of 5 liters, with a density of about 2 million of mites per liter. Mites were introduced in treatments 1, every two to three weeks, in the following dates: 21/9, 4/10, 16/10, 31/10, 17/11, 8/12, 27/12, 17/01, 30/01, 21/02, 14/03, 28/03 and 18/04. The dosage introduced each feeding day was 27 liters/ ha. To distribute the material a full spoon was released by hand (aprox 1.8 mls) in each plant.

In treatment 2, *Artemia* was introduced at a commercial dosage of 200 grams/ha (250,000 cysts per gram) in the same feeding dates than treatment 1. The material was spread applying a small amount (aprox. 0.07 g) at the top of a leave (on a medium-higher position) every 5 plants.

To evaluate the seasonal abundance of the populations of *N. tenuis* and the main pests a weekly sampling was performed. The number of nymphs and adults of *N. tenuis,* number of larvae and adults of thrips, number of larvae of *Tuta absoluta,* as well as number of nymphs, pupae and adults of whiteflies were counted every week on 3 leaves per plant (on a basal, medium and higher position) from a total of 10 randomly selected plants per treatment. The three rows of plants in the borders between treatments were kept as a buffer area and were not considered when sampling.

Means and standard errors of predators, thrips, *Tuta absoluta* and witheflies were calculated per treatment and sampling date. The numbers of *N. tenuis* were analyzed using an ANOVA per each sampling date and means separated using a Duncan test (SAS System 9.0).

### Results

Mean number (±SE) of nymphs and adults of *N. tenuis* counted in the three treatments in each sampling date are represented in figure 2. The populations were well established in 100% of the plants in the 3 treatments. The total mean number of individuals was 3.1, 2.1 and 1.2 in treatments 1, 2 and 3, respectively. No differences among treatments were observed the first two months, when temperatures were low (no heating system in the greenhouse), but from January, when temperatures slowly increased, *N. tenuis* developed higher populations in the treatments with in-crop food added, compared to the control. The highest numbers developed in the treatment with astigmatid mites, where between 2 to 3 times higher populations developed compared to the control, and up to 7 individuals/3 leaves were counted in May (fig. 2). The populations were significantly different among treatments on the dates: 17/01 (F= 0.03, p< 0.01), 30/01 (F=6.31, p=0.01), 14/02 (F= 15.25, p<0.001), 27/02 (F= 8.52, p< 0.01), 14/03 (F= 8.68, p<0.01), 28/03 (F=9.77, p<0.01), 9/04 (F=8.18, p<0.01), 18/04 (F=12.05, p<0.001), 27/04 (F3.48, p=0.05) and 9/05 (F=5.77, p= 0.01). Means of *N. tenuis* were higher in the treatment with prey mites compared to the control in all this dates, and were also higher than the treatment with *Artemia* in the dates 17/01 and 28/03. The means of *N. tenuis* in the treatment with *Artemia* were higher compared to the control in the dates 14/02, 27/02, 14/03, 9/04 and 18/04, but not significantly different than the control on 17/01, 30/01, 28/03, 27/04 and 9/05 (with P=0.05).

The infestations of *Tuta absoluta,* whiteflies and thrips were controlled successfully in all the treatments, but a higher number of larvae of *T. ab*soluta (figure 2b) and a higher population of whiteflies (figure 2c) developed in the control treatment compared to the treatments with added food for the predators.

### Conclusions

The introduction of the astigmatid mite *S. medanensis* gave an unexpected result allowing the development of two to three times higher populations of *N. tenuis* compared to the control plot (standard procedure of the growers). The populations of the predator when fed with the astigmatid mite were similar or even higher than the populations obtained when feeding the predators with cysts of *Artemia,* a factitious prey rather expensive that is used, as an in-crop food, mainly in high tech greenhouses in the north of Europe, where higher investments are made by growers to obtain a higher production per m².

The highest populations of *N. tenuis* developed from January, thanks to the feeding system with astigmatid mites, can guarantee a better control of the pests. Very commonly high infestations of thrips and whiteflies occur in the south of Spain from January, when the predators remain at low numbers. This affordable strategy with prey mites will offer a solution to the problem. The early built up of the populations from January also improves the preventive control of a major pest, the moth *Tuta absoluta,* that causes severe damages on tomatoes in the spring.

### Example 3. Introduction of astigmatid mites as food for Dicyphus bolivari in tomato crop (Comparative example, not being part of the invention)

### Objectives

To evaluate the use of astigmatid mites as in-crop factitious prey to improve the establishment of *Dicyphus bolivari* (Heteroptera: Miridae) in a greenhouse tomato crop. Specifically, to evaluate the introduction at the top of the plants of living populations of *Tyrolichus casei* (Acari: Astigmata), complemented or not with cysts of the brine shrimp *Artemia.* The aim is to compare these strategies with a standard procedure, without a feeding program, and also to compare it with the introduction of the eggs of the moth *Ephestia kuehniella,* which is a well-known factitious food with a high nutritive quality, used in most of the laboratory and industrial mass-rearing systems of predatory bugs produced in climatic chambers by biomanufacturers.

### Materials & methods

The trial was conducted in an experimental greenhouse in the south of Spain (Almeria) in the autumn-spring of 2018/19. The plastic greenhouse had 800 m², with the typical 'parral' structure of the area, with a flat roof, with zenithal and lateral windows to offer ventilation, all covered with insect proof nets, and without active climate control, which are the structures comprising 80% Almeria's greenhouses. A tomato crop of the variety Caniles was transplanted on October the 31^{th}, 2018, with a density of 1.5 plants/m². The predator *D. bolivari* was released in the seedlings two weeks before transplanting, with a rate of 1.5 individuals/plant. The same day when the predators were released a feeding was performed, introducing 10 gr of eggs of the moth *E. kuehniella* every 1,000 predators. This is a common practice by the growers to feed another mirid predator, *N. tenuis,* which is the most used species in the Mediterranean countries. Additionally, another feeding was performed using the same factitious food and dosage one week before transplanting.

After transplanting, four treatments were evaluated in the greenhouse based on a regular introduction at the top of the plants of different in-crop factitious prey:
(T1) *Tyrolichus casei* (Acari: Astigmata)
(T2) *T. casei* complemented with cysts of the brine shrimp *Artemia* (Branchiopoda: Artemiidae)
(T3) eggs of *Ephestia kuehniella*
(T4) Control without additional releases of in-crop food

Every treatment was applied in approximately 200 m². Two insect proof nets were established (from the ground to the roof), as barriers to separate physically the treatments 3 and 4.

The populations of the mite *T. casei* were produced and packaged in the facilities of Agrobio, with bran and vermiculite as a substrate, with a density between 1 and 2 million of mites per liter. Mites were introduced in treatments 1 and 2, every two to three weeks, in the following dates: 21/9, 4/10, 16/10, 31/10, 17/11, 8/12, 27/12, 17/01, 30/01, 21/02, 14/03, 28/03 and 18/04. The dosage introduced each feeding day was 33 liters/ ha. To distribute the material a full spoon was released by hand (approximately 2.2 mL) in each plant.

In treatment 2, 6.6 grams of *Artemia* (250,000 cysts/gram) (approximately 330 grams per hectare) were introduced as a complement to the astigmatid mites. The cysts of brine shrimps were homogeneously mixed with the populations of mites (10 grams *Artemia* / liter of astigmatid mites) and distributed together with the mites.

In treatment 3, 1.5 grams of *Ephestia kuehniella* (approximately 35,000 eggs/gram) (75 grams per hectare) were introduced every feeding day in the same dates than treatments 1 and 2. The material was spread applying a small amount (aprox. 0.025 g) at the top of a leave (on a medium-higher position) every 5 plants.

To evaluate the seasonal abundance of the populations of *D. bolivari* and the main pests a weekly sampling was performed. The number of nymphs and adults of *D. bolivari* were counted every week on 3 leaves per plant (on a basal, medium and higher position) from a total of 10 randomly selected plants per treatment.

Means and standard errors of predators, thrips and whiteflies were calculated per treatment and sampling date. The numbers of *D. bolivari* were analyzed using an ANOVA per each sampling date and means separated using a Duncan test (SAS System 9.0).

### Results

Mean number (±SE) of nymphs and adults of *D. bolivari* counted in the four treatments in each sampling date are represented in figure 3. The populations were well established in 100% of the plants in the 4 treatments. However, the numbers were very different, with a total mean number of individuals (every 3 leaves) of 1.8, 2.4, 2.0 and 0.5 in treatments 1, 2, 3 and 4, respectively. Despite the very late planting, the populations of predators were kept at high numbers during the winter in all the treatments with a feeding program, while the populations were lower in the control plot, as expected. The same low numbers of another miridae (*N. tenuis*) are counted in most of the tomato greenhouses in winter in the south of Spain, where no heating system is available, and no feeding program is applied. From February, when temperatures slowly increased, the populations developed higher numbers in the treatments with factitious prey. Surprisingly, the populations fed with mites were as abundant as the populations fed with the eggs of *Ephestia kuehniella,* and the highest numbers developed in treatment 2, with mites complemented with a small number of cysts of *Artemia.* In this treatment with mites mixed with *Artemia* more than 7 predators were counted every 3 leaves, 8 times higher than the population of *D. bolivari* counted in the control plants (fig. 3). The populations were significantly different among treatments on the dates:
19/11 (F= 3.6, p= 0.03), 04/12 (F=3.7, p=0.02), 15/02 (F= 12.6, p<0.001), 25/02 (F= 20.1, p< 0.0001) and 05/03 (F= 10.1, P<0.001). On November the 19^{th} means of *D. bolivari* were higher in the treatment with *E. kuehniella* (T3), compared both to the treatment with *Artemia* and prey mites (T2) and to the control (T4), but without differences compared to the treatment with only prey mites (T1). In December the 12^{th} means in the treatment with *E. kuehniella* were higher than the control, but without differences with the other treatments with in-crop food added, while there were no differences between T1, T2 and the control (T4). In February the 15^{th} means were higher in treatment with prey mites and *Artemia* (T2) compared to the other treatments, means with *E. kuheniella* were higher than the control and there were no differences between treatment with *E. kuheniella* and treatment with prey mites. In February the 25^{th} means in treatment with *Artemia* and prey mites (T2) were also higher than the other treatments, and T1 and T3 were higher than the control and without differences between them. In March the 3^{rd}, means in all the treatments with in-crop food were higher than the control, and means in T2 were higher than T3 but without differences with T1 (with P=0.05).

### Conclusions

The introduction of the astigmatid mite *T. casei* gave an unexpected result allowing the development of up to five times higher populations of *D. bolivari* compared to the control plot, and similar results compared to the populations fed with the very expensive eggs of *E. kuehniella.* Moreover, the populations fed with a mixture of the mites with a small percentage of cysts of *Artemia* were higher than the populations fed with the well-known high-quality eggs of *E. kuehniella.*

The establishment of an affordable feeding program based on releases of astigmatid mites, complemented or not with cysts of *Artemia,* can guarantee a conservation of higher numbers of individuals during winter, and favors the development of a high population at the end of winter. This can improve the control of the pests. Very commonly high infestations of thrips and whiteflies occur in the south of Spain from January, when the predators remain at low numbers. This affordable strategy with prey mites will offer a solution to the problem.

### Example 4. Selection of a strain of Orius laevigatus capable of feeding on pollen

### Objectives

To select a strain of *Orius laevigatus* for a better performance when fed pollen, and to compare its reproductive fitness feeding on pollen with two commercial populations (Agrobio and Koppert). This selection might involve a loss in the ability of this new strain to perform feeding prey, in contrast to feeding plant material like pollen. To evaluate if the selection compromised the performance of the predator when feeding prey, reproductive fitness feeding a factitious prey (*Ephestia* eggs) was also compared among the new selected strain and the commercial populations. In addition, the new elected strain was also compared with a strain acclimated, but not selected, to pollen over many generations to distinguish between adaptation (selection) and acclimation (phenotypic plasticity).

### Materials and methods

### Selection for pollen tolerance

Wild populations of *Orius laevigatus* were collected from natural flowering plants in different non-cultivated areas all over the Mediterranean basin. For each population, early fecundity (10 days) was assessed on an alternative diet consisting of finely ground dehydrated commercial honey bee pollen (hereafter pollen). Freshly emerged adults (<24 h old) from each population were separated. After 4 days during which mating and pre-oviposition period took place, adults were sexed and females isolated to test fecundity. At least 50 females were placed individually into small polypropylene recipients (45 mL) with ventilated lids with a piece of green bean pod end-sealed with paraffin wax as an egg-laying substrate, and pollen as food. Eggs counting was carried out every 2-3 days, switching females to a clean piece of bean pod and adding fresh food. Fecundity was assessed until day ten since mating (*F₁₀*). During this time, egg-laid bean pods were kept separately at low temperature (15°C). Once fecundity was recorded, the eggs from the females having shown the highest fecundity were selected. The offspring selected from each population made up the first step for the selection strain (hereafter POL).

Throughout the subsequent selection process, the selected strain was reared on a suboptimal diet, providing with pollen every 2-3 days, and with restricted (half the optimum) *Ephestia* eggs every week, in order to maintain zoophytophagy in balance.

From the initial selection, each selection cycle consisted on evaluating at least 200 females for their *F₁₀* feeding on pollen. Adults were changed from the suboptimal diet to pollen within 24 h after emergence. Three days after, females were isolated and tested for fecundity during 10 days. Then, the offspring from those females with a *F₁₀* value above the mean rate in the previous selection cycle was mixed together to initiate the next selection cycle. This procedure was followed for every subsequent selection cycle up to the sixth one (POL06). From then on, solely for the purpose of accelerating genetic gaining, a full-sibling family selection was carried out up to reach POL11. To do that, the offspring of the most fecund females was selected separately and then these second-generation families were assayed in the same way, isolating females to evaluate *F₁₀*, keeping bean pods with eggs at low temperature (15 °C). Finally, the best F1 families feeding on pollen were selected, mixing their offspring together to obtain the next selection step.

Along with this, an acclimated to pollen line was launched from a mixture of every wild population tested, with the aim of determining whether the genetic gain attained in our selected strain was due either to the breeding process or solely to this species' phenotypic plasticity to tolerate nutritious restriction. This acclimated population was maintained for the last three years (around 50 generations) in our laboratory with the suboptimal diet consisting in providing pollen every 2-3 days and restricted *Ephestia* eggs every week (see above), similarly to the selected strain but without suffering any selection.

### Life history traits

Biological characteristics (immature survival, longevity and lifetime fecundity) were evaluated both on *Ephestia* eggs and pollen. We compared the selected strain (POL11) with two commercial populations (Agrobio and Koppert), and the strain acclimated to pollen mentioned above (Acclimated).

Eggs in bean pods were counted and placed in plastic cups. Then 60-120 newly hatched nymphs (N1) per population were isolated in polypropylene 30 mL recipients with *Ephestia* eggs or pollen, according to the assessed treatment, and a piece of bean pod as source of moisture. Survival was monitored daily until adulthood.

Along with this, freshly emerged females and males of each population were allowed to mate for 4 days with *Ephestia* eggs or pollen. Then, females were isolated and fecundity was registered as above described with *Ephestia* eggs or pollen, in this case up to the females' death.

Biological characteristics among populations (selected strain, acclimated, and commercial populations) were analyzed by using ANOVA tests. When significant differences between populations were observed, means were separated using Tukey's HSD test. Females who lived less than ten days or didn't lay eggs were excluded from the analysis.

To calculate the life table parameters, the program rₘ 2.0 was used (Taberner A, Castañera P, Silvestre E, Bopazo J (1993) Comput Appl Biosci 9:535-5409), applied to the surviving data and the fecundity rates at different age classes, previously calculated, and considering a sex ratio of 1:1 based on the development observed during the bioassay. The data analysis was carried out using the bootstrap technique, performing 1000 repetitions, as suggested (Aragón-Sánchez M, Román-Fernández LR, Martinez-Garcia H et al (2018). BioControl 63: 785-794). Survival by ages (*Iₓ*), fecundity by ages (*mₓ*), as well as the following parameters were estimated from the life tables: intrinsic rate of natural increase (*rₘ*) and net reproduction rate (*R₀*).

### Results

Table 1 shows results of immature survival, longevity and lifetime fecundity for both nutritious treatments, *Ephestia* eggs and pollen. Immature survival on pollen was considerably higher in the selected strain (28%) compared to the reference populations (2-8%). By contrast, in the *Ephestia* treatment, survival was very similar among the Agrobio commercial population and the selected strain (71-76%), and faintly lower for the pollen-acclimated and Koppert commercial populations (52-57%).

In addition, when fed on pollen, we observed that our selected strain unquestionably showed statistically higher values both for longevity and fecundity, whose females lived around 10 days longer (31 days) than acclimated, commercial and wild strains (20-21 days), doubling the number of eggs laid during that time as compared to these reference populations (61 versus 27-31 eggs/female).

When we analyzed performance on the factitious prey *Ephestia* eggs (Table 1), no statistical differences among populations were found either in longevity or lifetime fecundity.

Finally, lifetable parameters are presented in Table 2. No significant differences were observed between selected and reference lines fed on *Ephestia* eggs, apart from the Koppert population, for intrinsic rate of natural increase (*rₘ*). However, a significant effect was found in the pollen treatment, with the improved strain POL11 showing the highest values (0.073 versus -0.042 to 0.010). Similarly, the selected strain had enhanced values of net reproduction rates (*R₀*) (8.5 versus 0.2 to 1.3).

**Table 1. Life history traits for several Orius laevigatus strains on both pollen and Ephestia eggs diets.**

| **Diet** | **Population** | **Nymphal survival (%)** | **Longevity** | **Total fecundity** |
|---|---|---|---|---|
| *Ephestia* eggs | POL11 | 75.6 | 28.3 ± 2.5 | 147.4 ± 13.9 |
| | Acclimated | 56.7 | 31.6 ± 2.8 | 149.2 ± 13.8 |
| | Agrobio | 70.9 | 27.4 ± 1.9 | 138.6 ± 14.5 |
| | Koppert | 51.8 | 26.5 ± 2.4 | 133.1 ± 16.7 |
| | *F* | | 1.1ns | 0.3ns |
| Pollen | POL11 | 27.8 | 30.5 ± 2.0 | 60.8 ± 3.8 |
| | Acclimated | 8.1 | 20.2 ± 2.2 | 27.3 ± 3.6 |
| | Agrobio | 8.0 | 21.0 ± 2.3 | 31.4 ± 6.1 |
| | Koppert | 1.7 | 20.4 ± 1.3 | 29.0 ± 2.9 |
| | *F* | | 8.91*** | 18.9*** |

Means (± SE) within a column and diet followed by the same letter are not significantly different (ANOVA followed by Tukey HDS test) ns: no significant, *** *p*<0.001*.*

**Table 2. Life table parameters [intrinsic rate of natural increase (rₘ) and net reproductive rate (R₀)] of several Orius laevigatus populations for two different nutritional treatments. Means (± SE) within a column and diet followed by the same lower case letter are not significantly different (ANOVA followed by Tukey HDS test, P>0.05).**

| **Diet** | **Population** | ***rₘ*** | ***R₀*** |
|---|---|---|---|
| Ephestia | POL11 | 0.157 ± 0.002 a | 55.79 |
| eggs | Acclimated | 0.147 ± 0.003 ab | 42.39 |
| | Agrobio | 0.155 ± 0.002 a | 49.25 |
| | Koppert | 0.138 ± 0.004 b | 36.34 |
| Pollen | POL11 | 0.073 ± 0.002 a | 8.49 |
| | Acclimated | 0.003 ± 0.005 b | 1.12 |
| | Agrobio | 0.010 ± 0.007 b | 1.26 |
| | Koppert | -0.042 ± 0.003 c | 0.24 |

### Conclusions

The new strain showed enhanced reproductive fitness feeding on pollen compared to commercial populations. This strain selected for better performance when fed a plant-produced material like pollen showed no trade-offs when feeding on prey, with no differences among the new strain and the commercially available populations in life-history values when feeding on the factitious prey.

In addition, the new selected strain also exhibited better performance when fed pollen than the acclimated strain. Therefore, the improvement was due to genetic gain rather than to phenotypic plasticity to tolerate nutritious restriction.

The superior ability of this new strain to better exploit pollen as food source will contribute to increase field performance as biological control agent, improving resilience under prey shortage, especially in crops with low pollen quantity or quality. In addition, commercial producers of *O*. *laevigatus* might reduce costs by complementing *Ephestia* eggs with pollen, less expensive, in mass-rearing.

### Example 5. Characterization of the ability of the new strain of Orius laevigatus to feed on a factitious prey

### Objectives

A new strain of *Orius laevigatus* was selected for better fitness when fed a plant-produced material, showing a superior ability to exploit non-prey food. This selection might involve a loss in the ability to reproduce on prey. This strain showed similar fitness than commercial populations when feeding on a highly nutritious prey, like *Ephestia* eggs. However, it was necessary to know whether the reproductive performance was maintained when fed a suboptimal factitious prey of poor nutritional quality.

To this aim, the reproductive performance of the new strain of *Orius laevigatus* feeding on an Astigmatid mite was assessed. The objective was to compare the longevity and fecundity of the new strain, selected for the trait 'better performance when fed pollen', with a standard commercial population of *O*. *laevigatus,* when provided with a suboptimal factitious prey, Astigmatid mites, as the main food source.

### Materials and methods

Biological characteristics of adults (longevity and lifetime fecundity) were evaluated on the diet based on Astigmatid mites, *Carpoglyphus lactis* produced and commercially sold by Agrobio (Powermite), which are packaged with bran and vermiculite as a substrate in cardboard bottles (density between 1-5 million mites per liter).

The selected strain (POL11) was compared with one commercial population (Agrobio).

Freshly emerged females and males of each population were allowed to mate for 4 days with excess of prey mites (Powermite). After mating and pre-oviposition period, adults were sexed and females isolated to test fecundity. Females were placed individually into small polypropylene recipients (45 mL) with ventilated lids with a piece of green bean pod end-sealed with paraffin wax as an egg-laying substrate, and excess of prey mites (Powermite) as food. Eggs counting was carried out every 2-3 days, switching females to a clean piece of bean pod and adding fresh food. Fecundity was assessed until the females' death.

Biological characteristics among populations (selected strain and commercial population) were analyzed by using ANOVA tests. When significant differences between populations were observed, means were separated using Tukey's HSD test. Females who didn't lay eggs were excluded from the analysis.

### Results

Data of longevity and lifetime fecundity of females of the commercial population (Agrobio) and the new strain (POL11) when fed Astigmatid mites (Powermite) are shown in figure 4. Both longevity and fecundity were significantly higher in the new strain, compared with the standard population. Longevity in the new strain was three times longer than that for the commercial population. More importantly, lifetime fecundity was two and a half higher in the new strain compared to that of the commercial population.

### Conclusions

The new strain exhibited better performance than the standard population when fed Astigmatid mites. This new strain of *O*. *laevigatus* was selected for the trait 'better performance with pollen as food'. Pollen being a plant material, the selection might involve a loss in the ability to exploit animal prey. Our aim was to check whether this loss was not too large, and the new strain was capable to maintain reproductive fitness when fed a factitious prey of poor nutritional quality like Astigmatid mites. Unexpectedly, not only was the fitness maintained, but the new strain performed much better when fed this suboptimal animal prey, compared to the standard commercially available *O. laevigatus.* Moreover, this was also surprising because the new strain didn't show better reproductive performance when feeding a highly nutritious factitious prey, *Ephestia* eggs (see Example 4). Adults of the new strain lived longer and laid more eggs when fed prey mites. The better performance suggests that the new strain will presumably respond better to supplemental food in the crop, consisting on Astigmatid mites. In addition, the commercial producers of *O*. *laevigatus* might reduce costs by complementing *Ephestia* eggs, used as factitious food for mass-rearing, with this less expensive food source in mass-rearing.

### Example 6. Introduction of astigmatid mites as food for the new strain of Orius laevigatus in a seedless variety of pepper crop

### Objectives

To evaluate the establishment of a new strain of *Orius laevigatus* in a seedless variety of pepper with low amount and/or poor quality of pollen, and to examine the capacity of this predator to control the main pest, the thrips *Frankliniella occidentalis,* in this greenhouse crop. The aim was to compare the new strain, that was selected for the trait 'better performance when fed with pollen', to a standard commercial population of *O. laevigatus.*

### Materials & methods

The trial was conducted in a commercial greenhouse in the south of Spain (Almeria) in the spring of 2020. The greenhouse was a typical 'parral' structure used in the Mediterranean area with aprox. 2,000 m². A pepper crop of the mini Capia variety 'Pepperito' was transplanted in February the 2^{on}. Two introductions of *O*. *laevigatus* were performed, each one with a dosage of 4 individuals/m², in March the 18^{th} and the 23^{rd.} Two treatments were evaluated:
- (1) Releases of standard population of *O*. *laevigatus*
- (2) Releases of the new strain of *O*. *laevigatus*

The treatments were separated by a net that was placed in the middle of the greenhouse from the ground to the roof. The predators of both treatments were fed with a regular program of introductions of factitious prey. In each treatment, one liter of a populations of the astigmatid mite *Carpoglyphus lactis* produced and commercially sold by Agrobio (Powermite), which are packaged with bran and vermiculite as a substrate in cardboard bottles (density between 1-5 million mites per liter), complemented with 10 grams of cysts of the brine shrimps *Artemia* (250,000 cysts/gram), were introduced in the same dates when the predators were released. Afterwards, one liter of population of the astigmatid mite *Suidasia medanensis,* commercially sold by Agrobio (Powerfood) (aprox. 1 million mites per liter), which is also packaged with vermiculite and bran as substrate, complemented with the same amount of *Artemia,* were introduced in each treatment in April the 8^{th}, 15^{th} and 29^{th}. The cysts of brine shrimps were homogeneously mixed with the populations of mites and distributed together with the mites. To distribute the material a small spoon was released by hand (aprox 0.5 mls) in each plant.

To evaluate the seasonal abundance of the populations of *O*. *laevigatus* and the main pests a weekly sampling was performed. The number of nymphs and adults of *O*. *laevigatus,* as well as the number of larvae and adults of thrips, were counted every week on 3 leaves (on a basal, medium and higher position) and 2 flowers per plant from a total of 20 randomly selected plants per treatment.

Means and standard errors of predators and thrips were calculated per treatment and sampling date. The numbers of *O*. *laevigatus* were analyzed using an ANOVA per each sampling date and means separated using a Duncan test (SAS System 9.0).

### Results

Mean number (±SE) of nymphs and adults of *O*. *laevigatus* counted in the two treatments in each sampling date are represented in figure 5. The populations were well established in 100% of the plants in the two treatments. The populations were significantly different between treatments on the dates: 6/04 (F= 4.08, p= 0.05), 15/04 (F=8.38, p<0.01) and 28/04 (F= 13.08, p<0.01). Means were different between treatments and significantly higher with the new strain compared to the control (with P=0.05).

The populations of thrips were successfully controlled in both treatments but were lower in the treatment with the new strain of the predator compared to the control.

### Conclusions

Both populations of *O*. *laevigatus* established in a mini Capia variety of peppers with the help of a regular program of introductions of factitious prey as complementary food. However, the new strain of *O*. *laevigatus,* that was selected for the trait 'better development with pollen as food', surprisingly performed better in this greenhouse crop that has no pollen, compared to the standard commercially available *O. laevigatus.* The new strain developed higher populations and offered a better control of the thrips. The better establishment of the new strain of *O*. *laevigatus* in crops without pollen offers the opportunity to set up efficient biocontrol programs to control the thrips in this mini Capia varieties, as well as in other crops without or with low quality of pollen.

### Example 7. Introduction of astigmatid mites as food for the new strain of Orius laevigatus in a cucumber crop

### Objectives

To evaluate the establishment of a new strain of *Orius laevigatus* in a cucumber crop, and to examine the capacity of this predator to control the main pest, the thrips *Frankliniella occidentalis,* in this greenhouse crop. The aim was to compare the new strain, that was selected for the trait 'better performance when fed with pollen', to a standard commercial population of *O*. *laevigatus.* This predator is commonly released in cucumbers like a 'chemical treatment' since no establishment is expected due to the lack of pollen of the crop.

### Materials & methods

The trial was conducted in a commercial greenhouse in the south of Spain (Almeria) in the spring of 2020. The greenhouse was a typical 'parral' structure used in the Mediterranean area with aprox. 3,000 m². A cucumber crop of a Dutch type variety Litoral (Rijk Zwaan) was transplanted in February the 27^{th}. Five introductions of *O*. *laevigatus* were performed, each one with a dosage of 2 individuals/m², in March the 18^{th} and the 25^{th}, and in April the 1^{st}, the 8^{th} and the 15^{th}. Two treatments were evaluated:
- (1) Releases of standard population of *O*. *laevigatus*
- (2) Releases of the new strain of *O*. *laevigatus*

The treatments were separated by a net that was placed in the middle of the greenhouse from the ground to the roof. The predators of both treatments were fed with a regular program of introductions of factitious prey. In each treatment, half a liter of a populations of the astigmatid mite *Suidasia medanensis,* commercially sold by Agrobio (Powerfood), which are packaged with bran and vermiculite as a substrate in cardboard bottles (aprox. 1 million mites per liter), complemented with 5 grams of cysts of the brine shrimps *Artemia* (250,000 cysts/gram), were introduced in the same dates when predators were introduced. The cysts of brine shrimps were homogeneously mixed with the populations of mites and distributed together with the mites. To distribute the material a small spoon was released by hand (aprox 0.33 mls) in every two plants.

To evaluate the seasonal abundance of the populations of *O*. *laevigatus* and the main pests a weekly sampling was performed. The number of nymphs and adults of *O*. *laevigatus,* as well as the number of larvae and adults of thrips, were counted every week on 3 leaves (on a basal, medium and higher position) and 2 flowers per plant from a total of 20 randomly selected plants per treatment.

Means and standard errors of predators and thrips were calculated per treatment and sampling date. The numbers of *O*. *laevigatus* were analyzed using an ANOVA per each sampling date and means separated using a Duncan test (SAS System 9.0).

### Results

Mean number (±SE) of nymphs and adults of *O*. *laevigatus* counted in the two treatments in each sampling date are represented in figure 6. The populations were significantly different between treatments on the dates: 21/04 (F= 27.05, p<0.001), 28/04 (F=4.58, p=0.03), 5/05 (F=12.67, p= 0.001) and 12/05 (F= 9.15, p<0.01). Means were different between treatments and significantly higher (with P=0.05) with the new strain, that developed between 2- 5 times higher populations compared to the control in the last 3 sampling dates.

The populations of thrips were successfully controlled in both treatments but were lower in the treatment with the new strain of the predator in the last two sampling dates. A decrease of the populations of thrips was obtained with the new predator strain from mid-April, where the pest populations decreased to half the numbers compared to the control in mid-May.

### Conclusions

Both populations of *O*. *laevigatus* were maintained in the cucumber crop thanks to the establishment of a feeding program. However, the new strain of *O*. *laevigatus,* that was selected for the trait 'better development with pollen as food', surprisingly performed better in this greenhouse crop that has no pollen, compared to the standard commercially available *O*. *laevigatus.* The new strain developed higher populations and offered a better control of the thrips. The better establishment of the new strain of *O*. *laevigatus* in crops without pollen offers the opportunity to set up efficient biocontrol programs to control the thrips in cucumbers, as well as in other crops without or with low quality of pollen.

### Example 8. Introduction of astigmatid mites as food for the new strain of Orius laevigatus in a chrysanthemum crop

### Objectives

To evaluate the establishment of a new strain of *Orius laevigatus* in a chrysanthemums crop, and to examine the capacity of this predator to control the main pest, the thrips *Frankliniella occidentalis,* in this greenhouse crop. The aim was to compare the new strain, selected for the trait 'better performance when fed with pollen', to a standard commercial population of O. *laevigatus.*

### Materials & methods

The trial was conducted in a commercial greenhouse situated in the Dutch province of Gelderland, between January and March 2020. The greenhouse was a typical high-tech Dutch glasshouse with artificial light and heating system with aprox. 3 hectares. Chrysanthemums are planted all year round (5 cycles per year), and following the common practice of the producers, the greenhouse was divided in plots of approx. 1,000 m², and different varieties of Chrysanthemums were planted in different plots with different starting dates, with a duration of each crop of 10 weeks. The trial was performed on two different plots of 1,000 m² with the same variety, both planted on January the 28^{th.}

One introduction of *O*. *laevigatus* was performed the first week after transplanting with a dosage of 10 individuals/m². Two treatments were evaluated (one in each plot) based on releases of different predator populations:
- (1) standard population of *O*. *laevigatus* commercially produced and sold by Agrobio
- (2) the new strain of *O*. *laevigatus*

The predators of both treatments were fed with a regular program of introductions of factitious prey. In each treatment, 2.5 liters of a populations of the astigmatid mite *Suidasia medanensis,* commercially sold by Agrobio (Powerfood) as in-crop food for predatory mites, were introduced once per week during all the trial. This prey mite populations, which are packaged with bran and vermiculite as a substrate in paper bags (aprox. 1 million mites per liter), were complemented in each release with 15 grams of cysts of the brine shrimps *Artemia* (250,000 cysts/gram). The cysts of brine shrimps were homogeneously mixed with the 2.5 l populations of mites and distributed together with the mites. To distribute the material an automatic device commercialized under the name Biospreader designed to distribute mites was used. This is based on a modified spin disc apparatus that is hang in the irrigation system and offers an even distribution of the material on the top of all the plants, so that a ratio of >100 factitious prey mites/plant were introduced each time.

To evaluate the seasonal abundance of the populations of *O*. *laevigatus* and the main pests a weekly sampling was performed. The number of nymphs and adults of *O*. *laevigatus,* as well as the number of larvae and adults of thrips, were counted every week on 10 randomly selected plants per treatment.

Means and standard errors of predators and thrips were calculated per treatment and sampling date. The numbers of *O*. *laevigatus* were analyzed using an ANOVA per each sampling date and means separated using a Duncan test (SAS System 9.0).

### Results

Mean number (±SE) of nymphs and adults of *O*. *laevigatus* counted in the two treatments in each sampling date are represented in figure 7. The populations were significantly different between treatments on weeks 4 (F= 6.82, p= 0.01), 5 (F=6.82, p=0.01) and 6 (F=4.71, p= 0.04). Means were different between treatments and significantly higher (with P=0.05) with the new strain, that developed between 4- 6 times higher populations compared to the control.

There was not a very high infestation of thrips during the winter period, and the populations of thrips were successfully controlled in both treatments. No additional chemical treatments were needed to control the pest. However, a chemical treatment was applied in week 7 to control another pest. Due to this treatment the predator populations disappeared in the control plot and decreased to low numbers in the treatment with the new strain, the last sampling week.

### Conclusions

Thanks to the in-crop release of astigmatid mites as factitious prey the populations of predators were established in both treatments. A first generation of the predator developed two weeks after the release (week 4 of the crop) and a second generation developed in week 6. The population of the new strain of *O*. *laevigatus,* selected by the trait 'better performance when fed with pollen', surprisingly established better in the crop (not yet with flowers) than the standard predator populations. Due to the higher populations of this new strain some predator populations survived after a chemical treatment while all the standard predator populations disappeared or drop to very low numbers.

These results offer, for the first time, the possibility to establish high populations of *O*. *laevigatus* in chrysanthemums crops. Current biocontrol programs in this crop are based on releases of predator mites, which offers a good control of the larvae of thrips. However, the predator mites cannot eat the adults of thrips, and often significant damages of the pest cannot be avoided. The lack of authorized chemical treatments, together with the very low accepted threshold of thrips populations in this crop, has brought a desperate situation for the growers. For this reason, researchers are evaluating the introduction of cysts of *Artemia* to establish *O*. *laevigatus,* as an important complementary tool, since this natural enemy can offer a control both of larvae and adults of thrips, as well as contribute to the control of other pests, like aphids. The better development of this new strain will offer a more robust biocontrol program and eventually may also decrease the needed release dosages, and so reduce the economic cost of the biocontrol.

### Example 10: Characterization of the ability of the new strain of Orius laevigatus to develop feeding on two astigmatid mites

### Objectives

A new strain of *Orius laevigatus* was selected for better fitness when fed a plant-produced material, showing a superior ability to exploit non-prey food. This selection might involve a loss in the ability to reproduce on prey. This strain showed similar fitness than commercial populations when feeding on a highly nutritious prey, like *Ephestia* eggs. In addition, it was tested that the new strain exhibited better performance than the standard population when fed the Astigmatid mite species *Carpoglyphus lactis* (Example 5). However, it was necessary to know whether the performance was maintained when fed on other Astigmatid mites species.

To this aim, the survival from egg to adult of the new strain of *Orius laevigatus* feeding on two Astigmatid mite species, *Tyrolichus casei* and *Aleuroglyphus ovatus,* was assessed. The objective was to compare the survival of the new strain, selected for the trait 'better performance when fed pollen', with a standard commercial population of O. *laevigatus,* when provided with a suboptimal factitious prey, Astigmatid mites, as the main food source.

### Materials and methods

Immature survival was evaluated on the diet based on Astigmatid mites, *Tyrolichus casei* and *Aleuroglyphus ovatus,* produced by Agrobio, which are packaged with bran and vermiculite as a substrate in cardboard bottles (density between 1-5 million mites per liter).

The selected strain (POL11) was compared with one commercial population (Agrobio).

Eggs in bean pods were counted and placed in plastic cups. Five replications of with at least 100 eggs each one were prepared for each mite prey and population. The bean pods with the egss were placed in polypropylene cups, and the Astigmatid mite species as diet and a piece of bean pod as source of moisture were changed every 2-3 days. Survival was monitored upon adult emergence.

Survival from egg to adult was analyzed by using an ANOVA test. When significant differences between populations were observed, means were separated using Tukey's HSD test.

### Results

Data of survival for egg to adult of the commercial population (Agrobio) and the new strain (POL11) when fed each Astigmatid mite species is shown in figure 8. Immature survival was around 40% for the standard population, much better than feeding on pollen with 8% survival (Example 4). Moreover, the immature survival was significantly higher in the new strain for both prey mites, reaching 52% feeding *Tyrolichus casei* and 76% feeding *Aleuroglyphus ovatus,* also much better than feeding on pollen (28% see example 4).

### Conclusions

The new strain exhibited better performance than the standard population when fed both Astigmatid mites. This new strain of *O*. *laevigatus* was selected for the trait 'better performance with pollen as food'. Pollen being a plant material, the selection might involve a loss in the ability to exploit animal prey. In a previous experiment (example 5) we checked that not only was this loss not too large, but the new strain outperformed the standard population when fed a factitious prey of poor nutritional quality like the Astigmatid mite *Carpoglyphus lactis.* In this experiment, we also found that the immature survival was significantly higher when feeding in two different Astignatid mites, *Tyrolichus casei* and *Aleuroglyphus ovatus,* in the new selected strain than in the standard commercial population.

The better performance suggests that the new strain will presumably respond better to supplemental food in the crop, consisting on Astigmatid mites. In addition, the commercial producers of *O*. *laevigatus* might reduce costs by complementing *Ephestia* eggs, used as factitious food for mass-rearing, with this less expensive food source in mass-rearing.

### Example 11: Characterization of the ability of Orius laevigatus to reproduce feeding on other prey mites

### Objectives

The standard *Orius laevigatus* strain was able to reproduce feeding on the Astigmatid mite *Carpoglyphus lactis* (see Example 5), but there is no information about whether is able to reproduce feeding on other Astigmatid mites, such as *Tyrolichus casei, Aleuroglyphus ovatus* and *Tyreophagus entomophagus.*

To this aim, the reproductive performance of a standard population of *Orius laevigatus* feeding on different Astigmatid mite species was assessed.

### Materials and methods

Lifetime fecundity was evaluated on the diet based on the Astigmatid mites, *Tyrolichus casei, Aleuroglyphus ovatus* and *Tyreophagus entomophagus* produced by Agrobio, which are packaged with bran and vermiculite as a substrate in cardboard bottles (density between 1-5 million mites per liter).

Freshly emerged females and males were allowed to mate for 4 days with excess of prey mites. After mating and pre-oviposition period, adults were sexed and females isolated to test fecundity. Females (40 for each mite species) were placed individually into small polypropylene recipients (45 mL) with ventilated lids with a piece of green bean pod end-sealed with paraffin wax as an egg-laying substrate, and excess of prey mites as food. Eggs counting was carried out every 2-3 days, switching females to a clean piece of bean pod and adding fresh food. Fecundity was assessed until the females' death.

Data were analyzed by using an ANOVA test. Females who didn't lay eggs were excluded from the analysis.

### Results

Data of lifetime fecundity of females of the commercial population (Agrobio) when fed different Astigmatid mite species are shown in figure 9. Adult fecundity feeding these Astigmatid species (26-33 eggs/female) was similar or higher than that feeding on *Carpoglyphus lactis* (21 eggs/female, example 5).

### Conclusions

*O*. *laevigatus* was able to reproduce feeding on the Astigmatid mites, *Tyrolichus casei, Aleuroglyphus ovatus* and *Tyreophagus entomophagus.*

### Example 12: Selection of a strain of Orius laevigatus tolerant to low temperatures

### Objectives

An important challenge facing biocontrol is extreme environmental conditions. Biological control agents (BCAs) can be especially sensitive to environmental fluctuations, because being ectotherms of small size, their physiological characteristics and life cycles are greatly affected by the variation in temperature. Importantly, temperature is probably the most significant environmental factor that influences the behaviour, distribution, development, survival and reproduction of an insect or mite. Thus, climatic conditions have a definitive impact on all species of insects and mites (pests and BCAs) present on a crop. However, the distinct response to environmental conditions between the BCAs and their prey will significantly affect BCAs establishment and reproduction rate. For example, the lower temperature threshold for O. *laevigatus* (11°C) is higher than that for its prey, *F. occidentalis* (8°C). This difference allows the development of the pest during the winter months inside the greenhouses, but not predator's development. Consequently, control failure is often observed in the coldest months. Similarly, in winter crops such as strawberry in unheated greenhouses, at the beginning of the crop the temperature is too low for BCA reproduction rate to be enough to reach the population levels that control pests. That is the case for *Orius* releases that have to be delayed until temperatures reach its lower threshold, with little time to establish and build up a population to control thrips. Therefore, the achievement of BCA strains tolerant to cold would make possible biocontrol under low temperature periods. Therefore the objective was to select a strain of *Orius laevigatus* for a better performance at temperatures close its lower development threshold (<15°C), and to compare its reproductive fitness at 15°C with two commercial populations (Agrobio and Koppert).

### Materials and methods

### Selection for cold tolerance

Wild populations of *Orius laevigatus* were collected from natural flowering plants in different non-cultivated areas all over the Mediterranean basin. For each population, early fecundity (10 days) was assessed at 15°C. Freshly emerged adults (<24 h old) from each population were separated. After 4 days during which mating and pre-oviposition period took place, adults were sexed and females isolated to test fecundity. At least 50 females were placed individually into small polypropylene recipients (45 mL) with ventilated lids with a piece of green bean pod end-sealed with paraffin wax as an egg-laying substrate, and *Ephestia* eggs as food. Eggs counting was carried out every 7 days, switching females to a clean piece of bean pod and adding fresh food. Fecundity was assessed until day 15 since mating (*F₁₅*). During this time, egg-laid bean pods were kept separately at low temperature (6°C). Once fecundity was recorded, the eggs from the females having shown the highest fecundity were selected. The offspring selected from each population made up the first step for the selection strain (hereafter COLD-PLUS).

From the initial selection, each selection cycle consisted on evaluating at least 200 females for their *F₁₅* at 15°C. Females were isolated and tested for fecundity during 15 days. Then, the offspring from those females with a *F₁₅* value above the mean rate in the previous selection cycle was mixed together to initiate the next selection cycle. This procedure was followed for every subsequent selection cycle, eventually obtaining the strain COLD PLUS.

Early fecundity *F₁₅* on *Ephestia* eggs among the selected strain (COLD PLUS) with two commercial populations (Agrobio and Koppert) was compared.

Data were analyzed by using an ANOVA test. When significant differences between populations were observed, means were separated using Tukey's HSD test. Females who lived less than 15 days or didn't lay eggs were excluded from the analysis.

### Results

Table 3 shows results of early fecundity (F₁₅) on *Ephestia* eggs at low temperature (15°C) for the selected population (COLD PLUS) and commercial populations as reference.

Fecundity was significantly higher for the selected population COLD PLUS (34.8 eggs/female) than for the commercial populations (19.3-19.5 eggs/female).

**Table 3. Early fecundity (F₁₅) at 15°C for several Orius laevigatus strains on Ephestia eggs.**

| **Population** | **Early fecundity** |
|---|---|
| Agrobio | 19.5 ± 1.4 a |
| Koppert | 19.3 ± 1.3 a |
| COLD PLUS | 34.8 ± 0.9 b |

Means (± SE) followed by the same letter are not significantly different (p>0.05, ANOVA followed by Tukey HDS test).

### Conclusions

The new strain showed enhanced reproductive fitness at temperatures close to its lowest thermal threshold (<15°C) compared to commercial populations.

### Example 13: Genetical characterization of two selected strains that perform better when eating astigmatid mites as a factitious prey

### Introduction

A study was performed to identify a molecular marker ligated to the genetically enhanced character expressed in the two selected strains of *Orius laevigatus*: (1) adapted to pollen (called Orius PLUS) and (2) adapted to lower temperatures (called Orius COLD-PLUS). More than 200 microsatellite markers were tested, also called SSRs (Single Sequence Repeat) markers. Only one of them has worked as expected, ligated to the phenotypes of interest.

### Experimental Procedures

Genomic DNA from individuals of the two selected strains, Orius PLUS and Orius COLD-PLUS and from individuals of a wild-type (WT) of *Orius laevigatus* population (used as a control) was isolated by E.Z.N.A.^{®} insect DNA kit (Omega Bio-Tek) following the manufacturer's instructions from individuals at adult stage, which were previously grinded during 5 minutes at 35Hz by a laboratory ball mill (Retsch MM400) with a 3mm of diameter tungsten sphere. The DNA of three single individuals of each population of *O. laevigatus* was tested with approximately 200 SSR markers designed by us. Only the SSR marker called RP02_F (5'-TACACACTTTGCCGTAAATCAGA-3') (SEQ ID NO: 1) and RP02_R (5' -CGTTGTATTGGCATGATATAGG-3') (SEQ ID NO: 2) drove a polymorphic amplification pattern, which was recognizable among improved strains. The RP02 SSR marker was first described by Duan et al. (PLOS One, 2017, 12(2): e0172513). In order to ensure this preliminary result, at least 250 single individuals from each *O*. *laevigatus* population were tested by PCR using 1 unit of VWR^{®} Red Taq DNA Polymerase, 1X Key Buffer, 3mM Cl₂Mg, 0.5mM of DNTPs, 10pM from each oligonucleotide, and 5-25ng genomic DNA per reaction. The PCR was running in an Eppendorf Mastercycler Pro according to the program shown in Figure 10.

After amplification, all samples were electrophoresed in a 1.2% agarose gel (v/w) previously dyed with 0.2% of GelRed^{®} Nucleic Acid Stain (v/v) in 1X Sodium borate Buffer (SB) and visualized directly by UV illumination in a gel imaging system (Bio-Rad Gel^{™} Doc XR+ System). Pictures were captured by Image Lab Software version 6.1.0 (Bio-Rad Laboratories, Inc.).

### Results

SSR markers in combination with RAPD markers have become an important tool to understand the molecular diversity and the genetic relationships within species, including inter- and intraspecific genetic variability. More than 200 primers were analyzed in the three *O. laevigatus* genotypes (3 individuals of each one). However, only 10 SSR makers were found polymorphic and associated to phenotypes. After that, 16 adults of each *O*. *laevigatus* population were utilized to test the 10 SSR markers chosen in the first analysis, and only one of them showed a polymorphic pattern associated to the phenotype under study. Finally, at least 250 individuals were assayed with the RP02 SSR marker, in order to study the genetic linkage between the RP02 SSR marker and the selected character in both improved strains of O. *laevigatus.* As it is shown in Figure 1, RP02 SSR marker resulted in Orius PLUS exhibiting one amplification product of PCR (800-820bp - bases of pairs), in the 97% of cases (Figure 11A), while in Orius COLD-PLUS the RP02 SSR marker produced three PCR products (1300, 600 and 450bp, approximately) in 100% of the cases (Figure 11B). On the other hand, in WT Orius both amplification patterns were segregating near to 50% of frequency (Figure 11C).

## Claims

1. A method for rearing a predator insect from the genus *Orius* on a crop comprising:
(i) providing the plant with at least a breeding population of the insect from the genus *Orius,*
(ii) providing the plant with a first source of food for the predator insect, wherein said first source of food comprises a population of an astigmatid mite, wherein said population of an astigmatid mite is not a population of *Tyrophagus putrescentiae*
and
allowing the predator insect to prey on the astigmatid mite,
wherein the astigmatid mite is a storage mite from a family selected from the group consisting of Acaridae, Carpoglyphidae, Glycyphagidae and Suidasiidae.

2. A method for controlling a pest in a crop comprising:
(i) providing the crop with at least a breeding population of an insect from the genus *Orius,* wherein said insect is a predator for said pest and
(ii) providing the crop with a first source of food for the predator insect, wherein said first source of food comprises a population of an astigmatid mite, wherein said population of an astigmatid mite is not a population of *Tyrophagus putrescentiae,*
wherein the astigmatid mite is a storage mite from a family selected from the group consisting of Acaridae, Carpoglyphidae, Glycyphagidae and Suidasiidae.

3. The method according to claims 1 or 2, wherein the storage mite is from a genus selected from the group consisting of *Acarus, Tyrophagus, Aleuroglyphus, Caloglyphus, Suidasia, Thyreophagus, Carpoglyphus, Glycyphagus, Tyrolichus* and *Lepidoglyphus.*

4. The method according to any one of claims 1 to 3, wherein the predator insect from the genus *Orius* is an insect from a strain from the species *Orius laevigatus* with increased tolerance to a diet based on a factitious prey, wherein said strain is obtained by a method comprising artificially selecting a population of *Orius laevigatus* for its tolerance to a diet based on pollen under a selective pressure, wherein said selective pressure comprises using pollen as the main source of food.

5. The method according to claim 4, wherein the artificial selection comprises:
(i) breeding a population of *Orius laevigatus* using pollen as the main source of food,
(ii) selecting the female individuals from the population used in step (i) that have a higher fecundity,
(iii) selecting the offspring from the female individuals selected in step (ii),
(iv) mixing the offspring selected in step (iii) to form a new population and
(v) repeating steps (i) through (iv) at least once.

6. The method according to any one of claims 4 or 5, wherein the strain from the species *Orius laevigatus* with increased tolerance to a factitious prey is **characterized in that** when the genome of the strain is amplified by PCR using a pair or primers of SEQ ID NO: 1 and SEQ ID NO: 2, a fragment of 780-840 base pairs is obtained in at least 70% of the individuals of the strain.

7. The method according to claim 3, wherein the predator insect from the *genus Orius* is an insect from a strain from the species *Orius laevigatus* with increased tolerance to a diet based on a factitious prey, wherein said strain is obtained by a method comprising artificially selecting a population of *Orius laevigatus* for its tolerance to low temperatures under a selective pressure, wherein said selective pressure comprises rearing the population of *Orius laevigatus* at a temperature lower than 20°C .

8. The method according to claim 7, wherein the artificial selection comprises:
(i) breeding a population of *Orius laevigatus* at a temperature lower than 20°C,
(ii) selecting the female individuals from the population used in step (i) that have a higher fecundity,
(iii) selecting the offspring from the female individuals selected in step (ii),
(iv) mixing the offspring selected in step (iii) to form a new population and
(v) repeating steps (i) through (iv) at least once.

9. The method according to any one of claims 7 or 8, wherein the strain from the species *Orius laevigatus* with increased tolerance to a factitious prey is **characterized in that** when the genome of the strain is amplified by PCR using the pair or primers of SEQ ID NO: 1 and SEQ ID NO: 2 the following fragments are obtained in at least 70% of the individuals of the strain: (i) a fragment of 1280-1360 bp, (ii) a fragment of 580-640 bp and (iii) a fragment of 420-480 bp.

10. The method according to any one of claims 1 to 9, further comprising providing the crop with a second source of food for the predator insect.

11. The method according to any one of claims 2 to 10, wherein the pest is selected from the group consisting of thrips, whiteflies, aphids and moths.

12. Use of an astigmatid mite for rearing a predator insect from the genus *Orius* on a crop, wherein said astigmatid mite is not *Tyrophagus putrescentiae,* wherein the astigmatid mite is a storage mite from a family selected from the group consisting of Acaridae, Carpoglyphidae, Glycyphagidae and Suidasiidae.

13. A composition comprising at least a breeding population of a predator insect from the genus *Orius* and a first source of food for said predator insect, wherein said first source of food comprises a population of an astigmatid mite, wherein said population of an astigmatid mite is not a population of *Tyrophagus putrescentiae* nor a population of *Carpoglyphus lactis,* wherein the breeding population of the predator insect and the first source of food are physically separated so that there is no contact between the population of the predator insect and the population of the astigmatid mite, and wherein the astigmatid mite is an storage mite from a family selected from the group consisting of Acaridae, Carpoglyphidae, Glycyphagidae and Suidasiidae.

14. Use of the composition according to claim 13 for controlling a pest in a crop, where the pest is a prey for the predator insect.

## Patentansprüche

1. Ein Verfahren zur Aufzucht eines Raubinsekts der Gattung *Orius* auf einer Kulturpflanze, umfassend:
(i) Versehen der Pflanze mit mindestens einer Zuchtpopulation des Insekts der Gattung *Orius*,
(ii) Versehen der Pflanze mit einer ersten Nahrungsquelle für das Raubinsekt, wobei die erste Nahrungsquelle eine Population einer Astigmatina-Milbe umfasst, wobei die Population einer Astigmatina-Milbe keine Population von *Tyrophagus putrescentiae* ist
und
Ermöglichen, dass das Raubinsekt die Astigmatina-Milbe als Beute fangen kann, wobei die Astigmatina-Milbe eine Vorratsmilbe von einer Familie ist, die ausgewählt ist aus der Gruppe bestehend aus Acaridae, Carpoglyphidae, Glycyphagidae und Suidasiidae.

2. Ein Verfahren zum Bekämpfen eines Schädlings auf einer Kulturpflanze, umfassend:
(i) Versehen der Kulturpflanze mit mindestens einer Zuchtpopulation eines Insekts der Gattung *Orius,* wobei das Insekt ein Raubinsekt für den Schädling ist, und
(ii) Versehen der Kulturpflanze mit einer ersten Nahrungsquelle für das Raubinsekt, wobei die erste Nahrungsquelle eine Population einer Astigmatina-Milbe umfasst, wobei die Population einer Astigmatina-Milbe keine Population von *Tyrophagus putrescentiae* ist,
wobei die Astigmatina-Milbe eine Vorratsmilbe von einer Familie ist, die ausgewählt ist aus der Gruppe bestehend aus Acaridae, Carpoglyphidae, Glycyphagidae und Suidasiidae.

3. Das Verfahren nach Anspruch 1 oder 2, wobei die Vorratsmilbe von einer Gattung stammt, die ausgewählt ist aus der Gruppe bestehend aus *Acarus*, *Tyrophagus*, *Aleuroglyphus*, *Caloglyphus*, *Suidasia*, *Thyreophagus*, *Carpoglyphus*, *Glycyphagus*, *Tyrolichus* und *Lepidoglyphus*.

4. Das Verfahren nach einem der Ansprüche 1 bis 3, wobei das Raubinsekt der Gattung *Orius* ein Insekt eines Stamms der Art *Orius laevigatus* mit erhöhter Toleranz gegenüber einer Ernährung auf Grundlage einer künstlichen Beute ist, wobei der Stamm mit einem Verfahren erhalten wird, das die künstliche Selektion einer Population von *Orius laevigatus* aufgrund ihrer Toleranz gegenüber einer Ernährung auf Grundlage von Pollen unter einem Selektionsdruck umfasst, wobei der Selektionsdruck die Verwendung von Pollen als die Hauptnahrungsquelle umfasst.

5. Das Verfahren nach Anspruch 4, wobei die künstliche Selektion umfasst:
(i) Aufzucht einer Population von *Orius laevigatus* unter Verwendung von Pollen als der Hauptnahrungsquelle,
(ii) Selektion weiblicher Individuen aus der in Schritt (i) verwendeten Population, die eine höhere Fruchtbarkeit aufweisen,
(iii) Selektion der Nachkommen der in Schritt (ii) selektierten weiblichen Individuen,
(iv) Mischen der in Schritt (iii) selektierten Nachkommen, um eine neue Population zu bilden, und
(v) mindestens einmaliges Wiederholen der Schritte (i) bis (iv).

6. Das Verfahren nach einem der Ansprüche 4 oder 5, wobei der Stamm der Art *Orius laevigatus* mit erhöhter Toleranz gegenüber einer künstlichen Beute **dadurch gekennzeichnet ist, dass**, wenn das Genom des Stammes mittels PCR unter Verwendung eines Paares oder von Primern aus SEQ ID NO:1 und SEQ ID NO:2 amplifiziert wird, in mindestens 70 % der Individuen des Stammes ein Fragment von 780 - 840 Basenpaaren erhalten wird.

7. Das Verfahren nach Anspruch 3, wobei das Raubinsekt der Gattung *Orius* ein Insekt eines Stamms der Art *Orius laevigatus* mit erhöhter Toleranz gegenüber einer Ernährung auf Grundlage einer künstlichen Beute ist, wobei der Stamm mit einem Verfahren erhalten wird, das die künstliche Selektion einer Population von *Orius laevigatus* aufgrund ihrer Toleranz gegenüber niedrigen Temperaturen unter einem Selektionsdruck umfasst, wobei der Selektionsdruck die Aufzucht der Population von *Orius laevigatus* bei einer Temperatur unter 20 °C umfasst.

8. Das Verfahren nach Anspruch 7, wobei die künstliche Selektion umfasst:
(i) Aufzucht einer Population von *Orius laevigatus* bei einer Temperatur unter 20 °C,
(ii) Selektion der weiblichen Individuen aus der in Schritt (i) verwendeten Population, die eine höhere Fruchtbarkeit aufweisen,
(iii) Selektion der Nachkommen der in Schritt (ii) selektierten weiblichen Individuen,
(iv) Mischen der in Schritt (iii) selektierten Nachkommen, um eine neue Population zu bilden, und
(v) mindestens einmaliges Wiederholen der Schritte (i) bis (iv).

9. Das Verfahren nach einem der Ansprüche 7 oder 8, wobei der Stamm der Art *Orius laevigatus* mit erhöhter Toleranz gegenüber einer künstlichen Beute **dadurch gekennzeichnet ist, dass**, wenn das Genom des Stammes mittels PCR unter Verwendung des Paares oder von Primern aus SEQ ID NO:1 und SEQ ID NO:2 amplifiziert wird, die folgenden Fragmente bei mindestens 70 % der Individuen des Stammes erhalten werden: (i) ein Fragment von 1280 - 1360 bp, (ii) ein Fragment von 580 - 640 bp und (iii) ein Fragment von 420 - 480 bp.

10. Das Verfahren nach einem der Ansprüche 1 bis 9, des Weiteren umfassend das Versehen der Kulturpflanze mit einer zweiten Nahrungsquelle für das Raubinsekt.

11. Das Verfahren nach einem der Ansprüche 2 bis 10, wobei der Schädling ausgewählt ist aus der Gruppe bestehend aus Thripsen, Weißen Fliegen, Blattläusen und Motten.

12. Verwendung einer Astigmatina-Milbe zur Aufzucht eines Raubinsekts der Gattung *Orius* auf einer Kulturpflanze, wobei die Astigmatina-Milbe nicht *Tyrophagus putrescentiae* ist, wobei die Astigmatina-Milbe eine Vorratsmilbe von einer Familie ist, die ausgewählt ist aus der Gruppe bestehend aus Acaridae, Carpoglyphidae, Glycyphagidae und Suidasiidae.

13. Eine Zusammensetzung, die mindestens eine Zuchtpopulation eines Raubinsekts der Gattung *Orius* und eine erste Nahrungsquelle für das Raubinsekt umfasst, wobei die erste Nahrungsquelle eine Population einer Astigmatina-Milbe umfasst, wobei die Population einer Astigmatina-Milbe weder eine Population von *Tyrophagus putrescentiae* noch eine Population von *Carpoglyphus lactis* ist, wobei die Zuchtpopulation des Raubinsekts und die erste Nahrungsquelle physisch voneinander getrennt sind, so dass kein Kontakt zwischen der Population des Raubinsekts und der Population der Astigmatina-Milbe besteht, und wobei die Astigmatina-Milbe eine Vorratsmilbe von einer Familie ist, die ausgewählt ist aus der Gruppe bestehend aus Acaridae, Carpoglyphidae, Glycyphagidae und Suidasiidae.

14. Verwendung der Zusammensetzung nach Anspruch 13 zum Bekämpfen eines Schädlings auf einer Kulturpflanze, wobei der Schädling ein Beutetier für das Raubinsekt ist.

## Revendications

1. Un procédé d'élevage d'un insecte prédateur du genre *Orius* sur une culture comprenant :
(i) la fourniture à la plante d'au moins une population reproductrice de l'insecte du genre *Orius,*
(ii) la fourniture à la plante d'une première source de nourriture pour l'insecte prédateur, dans laquelle ladite première source de nourriture comprend une population d'un acarien astigmatide, dans laquelle ladite population d'un acarien astigmatide n'est pas une population de *Tyrophagus putrescentiae*
et
permettant à l'insecte prédateur de s'attaquer à l'acarien astigmatide,
dans lequel l'acarien astigmatide est un acarien de stockage d'une famille sélectionnée parmi le groupe consistant en Acaridae, Carpoglyphidae, Glycyphagidae et Suidasiidae.

2. Un procédé de lutte contre un ravageur dans une culture comprenant :
(i) la fourniture à la culture d'au moins une population reproductrice d'un insecte du genre *Orius,* dans lequel ledit insecte est un prédateur dudit ravageur et
(ii) la fourniture à la culture d'une première source de nourriture pour l'insecte prédateur, dans laquelle ladite première source de nourriture comprend une population d'un acarien astigmatide, dans laquelle ladite population d'un acarien astigmatide n'est pas une population de *Tyrophagus putrescentiae,*
dans lequel l'acarien astigmatide est un acarien de stockage d'une famille sélectionnée parmi le groupe consistant en Acaridae, Carpoglyphidae, Glycyphagidae et Suidasiidae.

3. Le procédé selon les revendications 1 ou 2, dans lequel l'acarien de stockage est d'un genre sélectionné parmi le groupe consistant en *Acarus, Tyrophagus, Aleuroglyphus, Caloglyphus, Suidasia, Thyreophagus, Carpoglyphus, Glycyphagus, Tyrolichus et Lepidoglyphus*.

4. Le procédé selon l'une quelconque des revendications 1 à 3, dans lequel l'insecte prédateur du genre *Orius* est un insecte issu d'une souche de l'espèce *Orius laevigatus* dotée d'une tolérance accrue à un régime alimentaire basé sur une proie factice, dans lequel ladite souche est obtenue par un procédé comprenant la sélection artificielle d'une population d'Orius *laevigatus* pour sa tolérance à un régime alimentaire à base de pollen sous une pression sélective, dans lequel ladite pression sélective comprend l'utilisation de pollen comme source principale de nourriture.

5. Le procédé selon la revendication 4, dans lequel la sélection artificielle comprend :
(i) la reproduction d'une population d'Orius *laevigatus* à l'aide de pollen comme source principale de nourriture,
(ii) la sélection des individus femelles parmi la population utilisée à l'étape (i) qui ont une plus grande fécondité,
(iii) la sélection de la descendance issue des individus femelles sélectionnés à l'étape (ii),
(iv) le mélange de la descendance sélectionnée à l'étape (iii) pour former une nouvelle population et
(v) la répétition des étapes (i) à (iv) au moins une fois.

6. Le procédé selon l'une quelconque des revendications 4 ou 5, dans lequel la souche de l'espèce *Orius laevigatus* dotée d'une tolérance accrue à une proie factice est **caractérisée en ce que** lorsque le génome de la souche est amplifié par PCR à l'aide d'une paire ou d'amorces de SEQ ID NO: 1 et SEQ ID NO: 2, un fragment de 780-840 paires de bases est obtenu dans au moins 70 % des individus de la souche.

7. Le procédé selon la revendication 3, dans lequel l'insecte prédateur du *genre Orius* est un insecte issu d'une souche de l'espèce *Orius laevigatus* dotée d'une tolérance accrue à un régime alimentaire basé sur une proie factice, dans lequel ladite souche est obtenue par un procédé comprenant la sélection artificielle d'une population d'*Orius laevigatus* pour sa tolérance aux basses températures sous une pression sélective, dans lequel ladite pression sélective comprend l'élevage de la population d'*Orius laevigatus* à une température inférieure à 20 °C.

8. Le procédé selon la revendication 7, dans lequel la sélection artificielle comprend : (i) la reproduction d'une population d'Orius *laevigatus* à une température inférieure à 20 °C,
(ii) la sélection des individus femelles parmi la population utilisée à l'étape (i) qui ont une plus grande fécondité,
(iii) la sélection de la descendance issue des individus femelles sélectionnés à l'étape (ii),
(iv) le mélange de la descendance sélectionnée à l'étape (iii) pour former une nouvelle population et
(v) la répétition des étapes (i) à (iv) au moins une fois.

9. Le procédé selon l'une quelconque des revendications 7 ou 8, dans lequel la souche de l'espèce *Orius laevigatus* dotée d'une tolérance accrue à une proie factice est **caractérisée en ce que** lorsque le génome de la souche est amplifié par PCR à l'aide de la paire ou d'amorces de SEQ ID NO: 1 et SEQ ID NO: 2, les fragments suivants sont obtenus dans au moins 70 % des individus de la souche : (i) un fragment de 1280-1360 pb, (ii) un fragment de 580-640 pb et (iii) un fragment de 420-480 pb.

10. Le procédé selon l'une quelconque des revendications 1 à 9, comprenant en outre la fourniture à la culture d'une deuxième source de nourriture pour l'insecte prédateur.

11. Le procédé selon l'une quelconque des revendications 2 à 10, dans lequel le ravageur est sélectionné parmi le groupe consistant en les thrips, les aleurodes, les pucerons et les papillons de nuit.

12. Utilisation d'un acarien astigmatide pour l'élevage d'un insecte prédateur du genre *Orius* sur une culture, dans laquelle ledit acarien astigmatide n'est pas *Tyrophagus putrescentiae,* dans laquelle l'acarien astigmatide est un acarien de stockage d'une famille sélectionnée parmi le groupe consistant en Acaridae, Carpoglyphidae, Glycyphagidae et Suidasiidae.

13. Une composition comprenant au moins une population reproductrice d'un insecte prédateur du genre *Orius* et une première source de nourriture pour ledit insecte prédateur, dans laquelle ladite première source de nourriture comprend une population d'un acarien astigmatide, dans laquelle ladite population d'un acarien astigmatide n'est pas une population de *Tyrophagus putrescentiae* ni une population de *Carpoglyphus lactis,* dans laquelle la population reproductrice de l'insecte prédateur et la première source de nourriture sont physiquement séparées de sorte qu'il n'y ait pas de contact entre la population de l'insecte prédateur et la population de l'acarien astigmatide, et dans laquelle l'acarien astigmatide est un acarien de stockage d'une famille sélectionnée parmi le groupe consistant en Acaridae, Carpoglyphidae, Glycyphagidae et Suidasiidae.

14. Utilisation de la composition selon la revendication 13 pour la lutte contre un ravageur dans une culture, le ravageur étant une proie pour l'insecte prédateur.
